# EUROPÄISCHE PATENTANMELDUNG

(11) **EP 0 480 276 A2**
(43) Veröffentlichungstag der Anmeldung: **15.04.1992**
(21) Anmeldenummer: 91116648.6
(22) Anmeldetag: 30.09.1991
(51) Int. Cl.: C07D 215/14, B41M 5/136, B41M 5/30, C07D 215/18, C07D 215/20, C07D 215/12, C07D 277/64

(54) **Carbonylaminostyryle**

(30) Priorität: 11.10.1990 DE 4032208
(71) Anmelder: BAYER AG, 51368 Leverkusen (DE)
(72) Erfinder: Hassenrück, Karin, Dr., W-4000 Düsseldorf (DE); Raue, Roderich, Dr., W-5090 Leverkusen (DE)

(57) **Zusammenfassung**

Die neuen Carbonylaminostyryle der Formel (I) 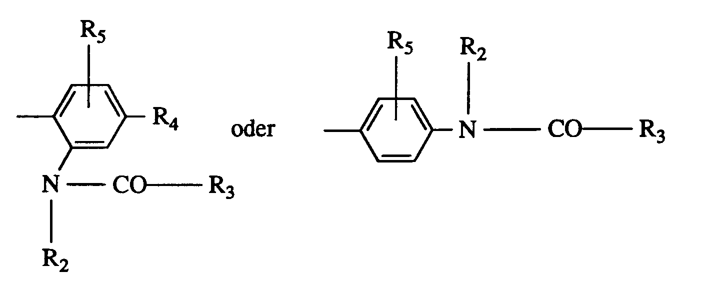
in welcher
die Substituenten R₁, A, B und Z die in der Beschreibung angegebene Bedeutung haben, eignen sich vorzüglich zur Herstellung druckkopierfähiger, thermoreaktiver oder elektrochromer Aufzeichnungsmaterialien.

## Beschreibung

Die vorliegende Erfindung betrifft neue Carbonylaminostyryle, Verfahren zu ihrer Herstellung und ihre Verwendung für druckkopierfähige, thermoreaktive oder elektrochrome Aufzeichnungsmaterialien.

Es wurden neue Cabonylaminostyryle der Formel 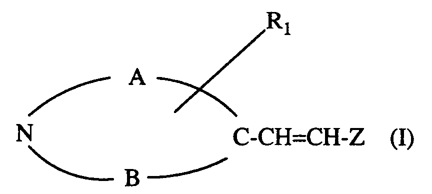
in welcher
- A: für die restlichen Glieder eines gegebenenfalls substituierten heterocyclischen Ringsystems, an welches gegebenenfalls ein weiterer Carbocyclus anelliert ist, steht,
- B: für eine Doppelbindung oder die Gruppierung =CH-CH= steht,
- R₁: für Wasserstoff, Alkyl, Alkenyl, Cycloalkyl, Alkoxy oder Halogen steht, und
- Z: für 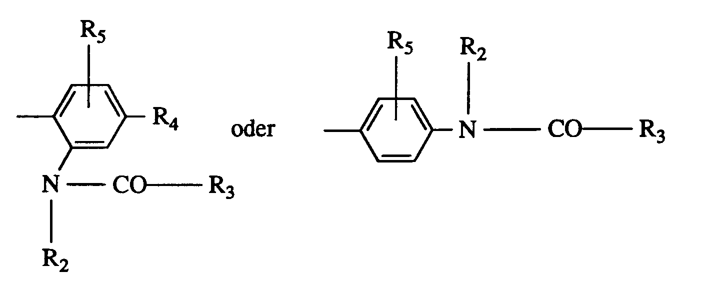 steht,
worin
- R₂: für Wasserstoff, Alkyl, Alkenyl, Cycloalkyl, Aralkyl, Aryl oder Hetaryl steht,
- R₃: für Wasserstoff, Alkyl Alkenyl, Cycloalkyl, Aralkyl, Aryl, Alkoxy, Dialkylamino, Alkylamino, Cycloalkylamino, Arylamino, Aralkylamino oder Hetaryl steht, oder
- R₂ und R₃: gemeinsam mit dem Stickstoffatom und dem Kohlenstoffatom, an welches sie gebunden sind einen gegebenenfalls substituierten 4- bis 8-gliedrigen Ring bilden, oder
- R₂ und R₅,: für den Fall, daß R₅ in ortho-Stellung zum Stickstoff steht, gemeinsam mit dem Stickstoffatom und dem Kohlenstoffatom an welches sie gebunden sind einen gegebenenfalls substituierten 5- bis 7-gliedrigen Ring bilden, und
- R₄ und R₅: unabhängig voneinander für Wasserstoff, Alkyl, Alkenyl, Cycloalkyl, Alkoxy oder Halogen stehen
gefunden.

Das durch A ergänzte heterocyclische System kann durch R₁ einfach oder mehrfach, gleich oder verschieden substituiert sein.

Alle vorstehend genannten Alkyl-, Alkenyl-, Cycloalkyl-, Alkoxy-, (Di)alkylamino-, Cycloalkylaminoreste können geradkettig oder verzweigt und gegebenenfalls einbis mehrfach, gleich oder verschieden substituierte sein. Die Aralkyl- und Aralkylaminoreste können sowohl im geradkettigen oder verzweigten Alkylteil als auch im Arylteil gegebenenfalls ein- oder mehrfach gleich oder verschieden substituiert sein.

Die Aryl- und Hetarylreste können gegebenenfalls ein- oder mehrfach gleich oder verschieden substituiert sein.

Alkylreste auch solche in z.B. Alkoxy, Alkylamino, Dialkylamino oder Aralkyl können bis zu 18 Kohlenstoffatomen aufweisen und z.B. durch Halogen, Alkoxy oder Cyano substituiert sein.

Alkenylreste können bis zu 18 Kohlenstoffatome aufweisen und z.B. durch Alkyl, Alkoxy, Halogen, Cyano oder Aryl substituiert sein.

Arylreste, auch solche in Aralkylgruppen, sind beispielsweise Phenyl, Naphthyl oder Anthracenyl, die z.B. durch Alkyl, Alkoxy, Halogen oder Cyano substituiert sein können.

Heterocyclische Reste können durch Alkyl, Alkoxy, Halogen oder Cyano substituiert sein können.

Bevorzugt sind Carbonylaminostyryle der Formel (I), in welcher
- A: für die restlichen Glieder eines 5- bis 7-gliedrigen, partiell gesättigten oder ungesättigten heterocyclischen Ringsystems steht, welches gegebenenfalls 1 oder 2 weitere Heteroatome aus der Reihe Sauerstoff, Schwefel, Stickstoff enthält, und an welches gegebenenfalls ein gesättigter oder ungesättigter 5- bis 7-gliedriger Carbocyclus anelliert ist,
- B: für eine Doppelbindung oder die Gruppierung =CH-CH=steht,
- R₁: für Wasserstoff, Alkyl mit 1 bis 8 Kohlenstoffatomen, welches gegebenenfalls einfach oder mehrfach durch Alkoxy mit 1 bis 6 Kohlenstoffatomen, Cyano, Hydroxy oder Halogen substituiert ist, Cycloalkyl mit 3 bis 8 Kohlenstoffatomen, Alkenyl mit 2 bis 6 Kohlenstoffatomen, Alkoxy mit 1 bis 6 Kohlenstoffatomen oder Halogen steht, und
- in Z:
- R₂: für Wasserstoff, Alkyl mit 1 bis 8 Kohlenstoffatomen, welches gegebenenfalls einfach oder mehrfach durch Alkoxy mit 1 bis 6 Kohlenstoffatomen, Hydroxy, Cyano oder Halogen substituiert ist, Alkenyl mit 2 bis 6 Kohlenstoffatomen, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Hydroxy oder Halogen substituiert ist, Aryl oder Aralkyl mit 1 bis 6 Kohlenstoffatomen im Alkylteil und jeweils 6 bis 10 Kohlenstoffatomen im Arylteil, welches jeweils gegebenenfalls im Arylteil einfach oder mehrfach durch Alkyl mit 1 bis 6 Kohlenstoffatomen oder Halogen substituiert ist oder für einen gesättigten oder ungesättigten 5- bis 7-gliedrigen heterocyclischen Ring, der 1 bis 4 Heteroatome aus der Reihe Sauerstoff, Schwefel, Stickstoff enthält und gegebenenfalls einfach oder mehrfach durch Alkyl mit 1 bis 6 Kohlenstoffatomen substituiert ist, und an welchen gegebenenfalls ein weiterer gesättigter oder ungesättigter 5- bis 6-gliedriger Carbocyclus anelliert ist, steht,
- R₃: für Wasserstoff, Alkyl oder Alkoxy mit jeweils 1 bis 18 Kohlenstoffatomen, das jeweils gegebenenfalls einfach oder mehrfach durch Alkoxy mit 1 bis 6 Kohlenstoffatomen, Cyano, Hydroxy oder Halogen substituiert ist, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Hydroxy oder Halogen substituiert ist, Aryl oder Aralkyl mit 1 bis 6 Kohlenstoffatomen im Alkylteil und jeweils 6 bis 10 Kohlenstoffatomen im Arylteil, welches jeweils gegebenenfalls im Arylteil einfach oder mehrfach durch Alkyl mit 1 bis 6 Kohlenstoffatomen oder Halogen substituiert ist, Alkenyl mit 2 bis 6 Kohlenstoffatomen, Dialkylamino oder Alkylamino mit jeweils 1 bis 18 Kohlenstoffatomen in den jeweiligen Alkylteilen, Cycloalkylamino mit 3 bis 8 Kohlenstoffatomen, Arylamino oder Aralkylamino mit 1 bis 6 Kohlenstoffatomen im Alkylteil und jeweils 6 bis 10 Kohlenstoffatomen in den jeweiligen Arylteilen, welches jeweils gegebenenfalls einfach oder mehrfach im Arylteil durch Alkyl mit 1 bis 6 Kohlenstoffatomen oder Halogen substituiert ist, oder für einen gesättigten oder ungesättigten 5- bis 7-gliedrigen heterocyclischen Ring, der 1 bis 4 Heteroatome wie Sauerstoff, Schwefel oder Stickstoff enthält und gegebenenfalls einfach oder mehrfach durch Alkyl mit 1 bis 6 Kohlenstoffatomen substituiert ist, steht, oder
- R₂ und R₃: gemeinsam mit dem Stickstoffatom bzw. dem Kohlenstoffatom an welches sie gebunden sind einen gesättigten oder ungesättigten 4- bis 8-gliedrigen Ring bilden, der gegebenenfalls einfach oder mehrfach durch Alkyl mit 1 bis 6 Kohlenstoffatomen un/oder Oxo substituiert ist, oder
- R₂ und R₅,: für den Fall, daß R₅ in ortho-Stellung zum Stickstoff steht, gemeinsam mit dem Stickstoffatom bzw. dem Kohlenstoffatom an welches sie gebunden sind einen gesättigten oder ungesättigten 5- bis 7-gliedrigen Ring bilden, der gegebenenfalls einfach oder mehrfach durch Alkyl mit 1 bis 6 Kohlenstoffatomen substituiert ist, und
- R₄ und R₅: unabhängig voneinander für Wasserstoff, Alkyl mit 1 bis 18 Kohlenstoffatomen, das gegebenenfalls einfach oder mehrfach durch Alkoxy mit 1 bis 6 Kohlenstoffatomen, Cyano, Hydroxy oder Halogen substituiert ist, Alkenyl mit 2 bis 6 Kohlenstoffatomen, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Cyano oder Halogen substituiert ist, Alkoxy mit 1 bis 18 Kohlenstoffatomen oder Halogen stehen.

Besonders bevorzugt sind Carbonylaminostyryle der Formel (I),
in welcher
- A: für die restlichen Glieder eines 5- bis 6-gliedrigen, partiell gesättigten oder ungesättigten heterocyclischen Ringsystems steht, welches gegebenenfalls 1 oder 2 weitere Heteroatome aus der Reihe Sauerstoff, Schwefel,Stickstoff enthält, und an welches gegebenenfalls ein ungesättigter 6-gliedriger Carbocyclus anelliert ist,
- B: für eine Doppelbindung oder die Gruppierung =CH-CH= steht,
- R₁: für Wasserstoff, Alkyl mit 1 bis 6 Kohlenstoffatomen, das gegebenenfalls 1-bis 3-fach durch Alkoxy mit 1 bis 4 Kohlenstoffatomen, Cyano, Hydroxy, Fluor, Chlor oder Brom substituiert ist, Cycloalkyl mit 3 bis 7 Kohlenstoffatomen, Alkenyl mit 2 bis 4 Kohlenstoffatomen, Alkoxy mit 1 bis 4 Kohlenstoffatomen,Fluor, Chlor oder Brom steht, und
- in Z:
- R₂: für Wasserstoff, Alkyl mit 1 bis 6 Kohlenstoffatomen, das gegebenenfalls 1- bis 3-fach durch Alkoxy mit 1 bis 4 Kohlenstoffatomen, Hydroxy, Cyano, Fluor, Chlor oder Brom substituiert ist, Alkenyl mit 2 bis 4 Kohlenstoffatomen, Cycloalkyl mit 3 bis 7 Kohlenstoffatomen, welches gegebenenfalls 1- bis 3-fach durch Alkoxy oder Alkyl mit jeweils 1 bis 4 Kohlenstoffatomen, Hydroxy, Fluor, Chlor oder Brom substituiert ist, Phenyl, Naphthyl, Benzyl oder Phenethyl, welches jeweils gegebenenfalls 1 bis 3-fach im Phenyl- bzw. Naphthylteil durch Alkyl mit 1 bis 4 Kohlenstoffatomen, Fluor, Chlor oder Brom substituiert ist, oder für einen gesättigten oder ungesättigten 5- oder 6-gliedrigen heterocyclischen Ring, der 1 bis 3 Heteroatome aus der Reihe Sauerstoff, Schwefel, Stickstoff enthält, und an welchen gegebenenfalls ein weiterer ungesättigter 5- bis 6-gliedriger Carbocyclus anelliert ist, steht,
- R₃: für Wasserstoff, Alkyl oder Alkoxy mit jeweils 1 bis 18 Kohlenstoffatomen, das jeweils gegebenenfalls 1- bis 3-fach durch Alkoxy mit 1 bis 4 Kohlenstoffatomen, Cyano, Hydroxy, Fluor, Chlor oder Brom substituiert ist, Cycloalkyl mit 3 bis 7 Kohlenstoffatomen, das gegebenenfalls 1- bis 3-fach durch Alkyl oder Alkoxy mit jeweils 1 bis 4 Kohlenstoffatomen, Hydroxy, Fluor, Chlor oder Brom substituiert ist, Phenyl, Naphthyl, Benzyl oder Phenethyl welches jeweils gegebenenfalls im Phenyl- bzw. Naphthylteil 1- bis 3-fach durch Alkyl mit 1 bis 4 Kohlenstoffatomen, Fluor, Chlor oder Brom substituiert ist, Alkenyl mit 2 bis 4 Kohlenstoffatomen, Dialkylamino oder Alkylamino mit jeweils 1 bis 18 Kohlenstoffatomen in den jeweiligen Alkylteilen, Cycloalkylamino mit 3 bis 7 Kohlenstoffatomen, Phenylamino, Naphthylamino, Benzylamino oder Phenethylamino welches jeweils gegebenenfalls 1- bis 3-fach im Phenyl- bzw. Naphthylteil durch Alkyl mit 1 bis 4 Kohlenstoffatomen, Fluor, Chlor oder Brom substituiert ist, oder für einen gesättigten oder ungesättigten 5- bis 7-gliedrigen heterocyclischen Ring, der 1 bis 3 Heteroatome aus der Reihe Sauerstoff, Schwefel, Stickstoff enthält, und 1- bis 4-fach durch Alkyl mit 1 bis 4 Kohlenstoffatomen substituiert ist, steht, oder
- R₂ oder R₃: gemeinsam mit dem Stickstoffatom bzw. dem Kohlenstoffatom an welches sie gebunden sind einen gesättigten oder ungesättigten 4-bis 8-gliedrigen Ring bilden, der gegebenenfalls 1- bis 6-fach durch Alkyl mit 1 bis 4 Kohlenstoffatomen und/oder Oxo substituiert ist, oder
- R₂ und R₅,: für den Fall, daß R₅ in ortho-Stellung zum Stickstoff steht, gemeinsam mit dem Stickstoffatom bzw. dem Kohlenstoffatom an welches sie gebunden sind einen gesättigten oder ungesättigten 5- bis 7-gliedrigen Ring bilden, der gegebenenfalls 1- bis 3-fach durch Alkyl mit 1 bis 4 Kohlenstoffatomen substituiert ist, und
- R₄ und R₅: unabhängig voneinander für Wasserstoff, Alkyl mit 1 bis 16 Kohlenstoffatomen, das gegebenenfalls 1- bis 3-fach durch Alkoxy mit 1 bis 4 Kohlenstoffatomen, Cyano, Hydroxy oder Halogen substituiert ist, Alkenyl mit 2 bis 4 Kohlenstoffatomen, Cycloalkyl mit 3 bis 7 Kohlenstoffatomen, das gegebenenfalls 1- bis 3-fach durch Alkyl oder Alkoxy mit jeweils 1 bis 4 Kohlenstoffatomen, Cyano oder Halogen substituiert ist, Alkoxy mit 1 bis 16 Kohlenstoffatomen, Fluor, Chlor oder Brom stehen.

Ganz besonders bevorzugt sind Carbonylaminostyryle der Formel (I),
in welcher 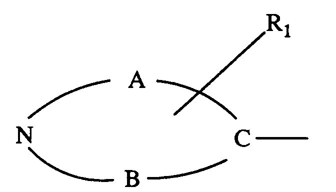
für 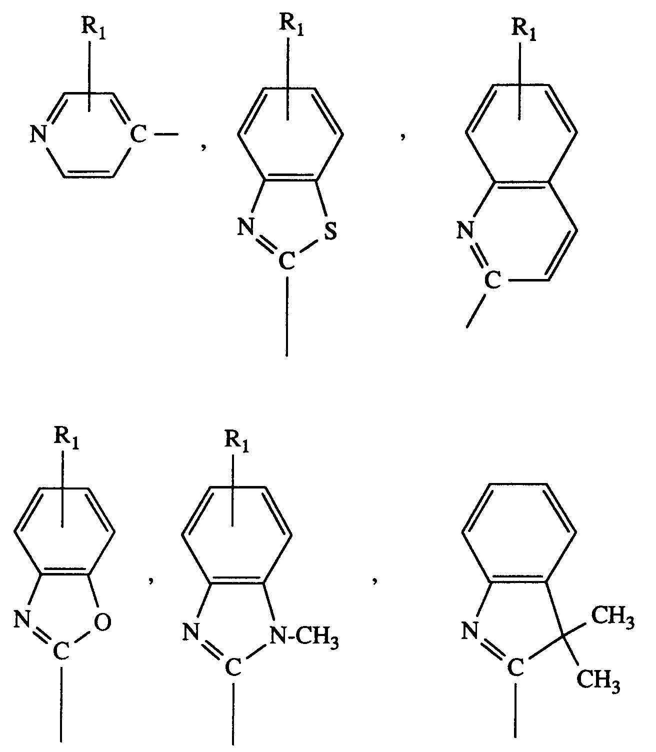
- R₁: für Wasserstoff, Alkyl mit 1 bis 4 Kohlenstoffatomen, das gegebenenfalls durch Methoxy, Ethoxy, Cyano, Hydroxy oder Chlor substituiert ist, Cyclopentyl, Cyclohexyl, Vinyl, Allyl, Methoxy, Ethoxy, n-Propoxy oder Chlor steht, und
- in Z:
- R₂: für Wasserstoff, Alkyl mit 1 bis 4 Kohlenstoffatomen, das gegebenenfalls durch Methoxy, Ethoxy, Cyano, Hydroxy oder Chlor substituiert ist, Cyclopentyl, Cyclohexyl,Vinyl, Allyl; für Phenyl oder Benzyl welches jeweils gegebenenfalls im Phenylteil durch Methyl, Methoxy, Cyano oder Chlor substituiert ist, oder für Pyridyl, Pyrimidyl, Benzimidazolyl, Benzoxazolyl, Benzthiazolyl oder Chinolyl steht,
- R₃: für Wasserstoff, Alkyl oder Alkoxy mit jeweils 1 bis 18 Kohlenstoffatomen, das jeweils gegebenenfalls durch Methoxy, Ethoxy, Cyano, Hydroxy oder Chlor substituiert ist; für Cyclopentyl, Cyclohexyl, Phenyl, Benzyl oder Naphthyl, welches jeweils gegebenenfalls durch Methyl, Methoxy, Cyano oder Chlor substituiert ist; für Vinyl, Allyl; Dialkylamino oder Alkylamino mit jeweils 1 bis 18 Kohlenstoffatomen in den jeweiligen Alkylteilen, Cyclopentylamino, Cyclohexylamino, Phenylamino, oder für Pyridyl, Pyrimidyl, Thienyl, Furyl, Pyrrolyl, Pyrazinyl, 1-Pyrrolidyl oder 1-Piperidyl steht, wobei die heterocyclischen Reste jeweils gegebenenfalls durch 1 bis 4 Methylgruppen substituiert sind, oder
- R₂ und R₃: gemeinsam mit dem Stickstoffatom, bzw. dem Kohlenstoffatom an welches sie gebunden sind, einen 5- oder 6-gliedrigen gesättigten oder ungesättigten Ring bilden, der gegebenenfalls 1- bis 6-fach durch Methyl und/oder Oxo substituiert ist, oder
- R₂ und R₅,: für den Fall, daß R₅ in ortho-Stellung zum Stickstoff steht, gemeinsam mit dem Stickstoffatom bzw. dem Kohlenstoffatom an welches sie gebunden sind einen gesättigten oder ungesättigten 5- bis 6-gliedrigen Ring bilden, der gegebenenfalls 1- bis 3-fach durch Methyl substituiert ist,
- R₄: für Wasserstoff, Alkyl mit 1 bis 14 Kohlenstoffatomen, das gegebenenfalls durch Methoxy, Ethoxy, Cyano, Hydroxy oder Chlor substituiert ist, Cyclopentyl, Cyclohexyl, Vinyl, Allyl, Alkoxy mit 1 bis 14 Kohlenstoffatomen oder Chlor steht, und
- R₅: für Wasserstoff, Alkyl oder Alkoxy mit jeweils 1 bis 14 Kohlenstoffatomen steht.

Insbesondere bevorzugt sing Carbonylaminostyryle der Formel (I),
in welcher 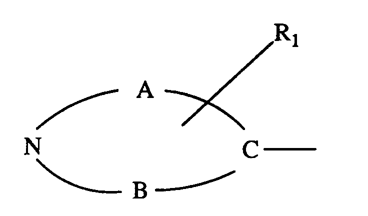
für 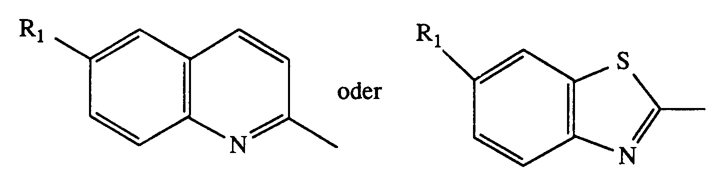
steht,
- R₁: für Wasserstoff, Methyl, Ethyl, Hydroxymethyl, Chlormethyl, Methoxymethyl, Chlorethyl, Cyanomethyl, Hydroxyethyl, Methoxy, Ethoxy oder Chlor steht,
- in Z:
- R₂: für Wasserstoff, Methyl, Ethyl, n-Propyl, i-Propyl, n-Butyl, s-Butyl, i-Butyl, t-Butyl, Hydroxymethyl oder Hydroxyethyl steht,
- R₃: für Wasserstoff, Methyl, Ethyl, Propyl, Hexyl, Heptyl, Octyl, 2-Methylpropyl, Nonyl, Decyl, Dodecyl, Hexadecyl, Octadecyl, Cyclopentyl, Cyclohexyl, Benzyl, Phenyl, Methoxy, Ethoxy, Propoxy, Butoxy, Pentoxy, Hexoxy, Heptoxy, Octoxy, 2-Ethylhexoxy, 1,3-Dimethylbutoxy, 1-Ethylpropoxy, 3,5,5-Trimethylhexoxy, Dodecyloxy, Hexadecyloxy, Octadecyloxy, Dimethylamino, Diethylamino, Dipropylamino, Dibutylamino, Diisopropylamino, Di(2-ethylbutyl)amino, Di(2-methylpropyl)amino, 1-Methylpropanamino, 1,1-Dimethylethanamino, Octylamino, Decylamino, Dodecylamino, Hexadecylamino, Octadecylamino, Cyclobutylamino, Cyclopentylamino, Cyclohexylamino steht,
- R₄: für Wasserstoff, Methyl, Ethyl, Propyl, Butyl, Hexyl, Methoxy, Ethoxy, Propoxy, Butoxy, Hexoxy, Octoxy, Decyloxy, Dodecyloxy oder Chlor steht, und
- R₅: für Wasserstoff, Methyl, Ethyl, Propyl, Butyl, Hexyl, Methoxy, Ethoxy, Propoxy, Butoxy, Hexoxy, Octoxy, Decyloxy, Dodecyloxy steht.

Insbesondere bevorzugt sind Carbonylaminostyryle der Formeln 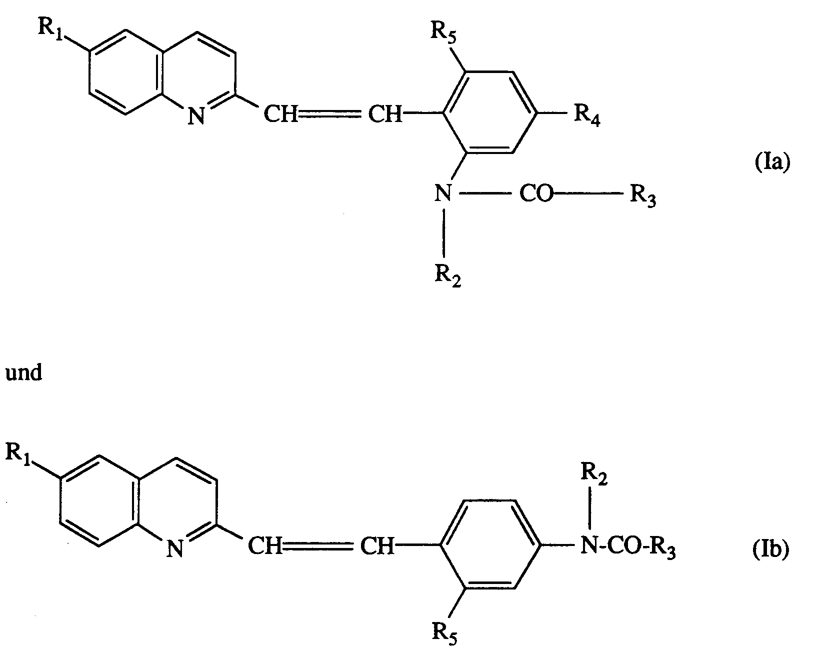
in welchen
- R₁: für Wasserstoff, Methyl, Ethyl, Methoxy, Ethoxy oder Chlor steht,
- R₂: für Wasserstoff, Methyl, Ethyl, n-Propyl, i-Propyl, n-Butyl, s-Butyl, i-Butyl oder t-Butyl steht,
- R₃: für Wasserstoff, Methyl, Ethyl, Propyl, Hexyl, Heptyl, Octyl, 2-Methylpropyl, Nonyl, Decyl, Dodecyl, Hexadecyl, Octadecyl, Cyclopentyl, Cyclohexyl, Benzyl, Phenyl, Methoxy, Ethoxy, Propoxy, Butoxy, Pentoxy, Hexoxy, Heptoxy, Octoxy, 2-Ethylhexoxy, 1,3-Dimethylbutoxy, 1-Ethylpropoxy, 3,5,5-Trimethylhexoxy, Dodecyloxy, Hexadecyloxy, Octadecyloxy, Dimethylamino, Diethylamino, Dipropylamino, Dibutylamino, Diisopropylamino, Di(2-ethylbutyl)amino, Di(2-methylpropyl)amino, 1-Methylpropanamino, 1,1-Dimethylethanamino, Octylamino, Decylamino, Dodecylamino, Hexadecylamino, Octadecylamino, Cyclobutylamino, Cyclopentylamino, Cyclohexylamino steht,
- R₄: für Wasserstoff, Methyl, Ethyl, Propyl, Butyl, Hexyl, Methoxy, Ethoxy, Propoxy, Butoxy, Hexoxy, Octoxy, Decyloxy, Dodecyloxy oder Chlor steht, und
- R₅: für Wasserstoff, Methyl, Ethyl, Propyl, Butyl, Hexyl, Methoxy, Ethoxy, Propoxy, Butoxy, Hexoxy, Octoxy, Decyloxy, Dodecyloxy steht.

Die neuen Carbonylaminostyryle der Formel (I) können hergestellt werden, indem man
A) Verbindungen der Formel (II) 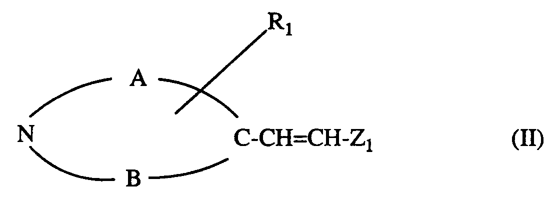 in welcher
   A,B und R₁ die oben angegebene Bedeutung haben, und
   Z₁ für 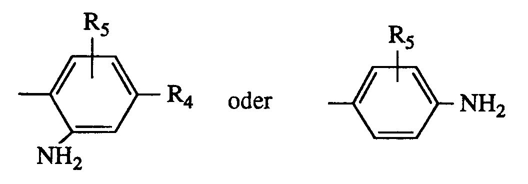 steht
   worin R₄ und R₅ die oben angegebene Bedeutung haben,
   entweder
   a) mit Verbindungen der Formel 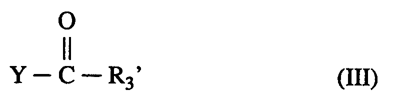 in welcher
      - Y: für Halogen, insbesondere für Chlor oder Brom steht, und
      - R₃': für Alkyl, Alkenyl, Cycloalkyl, Aralkyl, Aryl oder Hetaryl, insbesondere für Alkyl mit 1 bis 18 Kohlenstoffatomen, das gegebenenfalls einfach oder mehrfach durch Alkoxy mit 1 bis 6 Kohlenstoffatomen, Cyano, Hydroxy oder Halogen substituiert ist, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Hydroxy oder Halogen substituiert ist, Aryl oder Aralkyl mit 1 bis 6 Kohlenstoffatomen im Alkylteil und jeweils 6 bis 10 Kohlenstoffatomen im Arylteil, welches jeweils gegebenenfalls im Arylteil einfach oder mehrfach durch Alkyl mit 1 bis 6 Kohlenstoffatomen, Hydroxyl oder Halogen substituiert ist, Alkenyl mit 2 bis 6 Kohlenstoffatomen, oder für einen gesättigten oder ungesättigten 5-bis 7-gliedrigen heterocyclischen Ring, der 1 bis 4 Heteroatome wie Sauerstoff, Schwefel oder Stickstoff enthält und gegebenenfalls einfach oder mehrfach durch Alkyl mit 1 bis 6 Kohlenstoffatomen substiutiert ist, steht,
      acyliert, oder
   b) mit Chlorameisensäureestern zu den Verbindungen der Formel 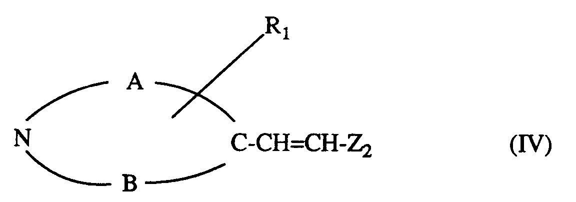 in welcher Z₂ für 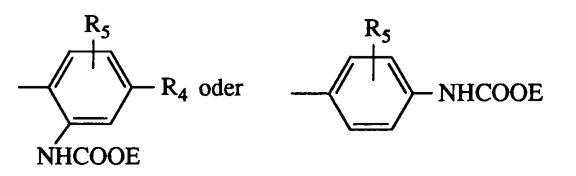 steht,
      worin
      R₄ und R₅ die oben angegebene Bedeutung haben,
      und E für Methyl, Ethyl oder Phenyl steht,
      A, B und R₁ die oben angegebene Bedeutung haben, umsetzt, und die so erhaltenen Verbindungen der Formel (IV) gegebenenfalls weiter mit
      α) Aminen der Formel 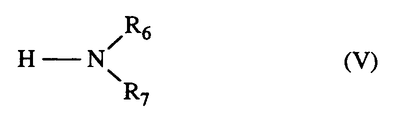 in welcher
         - R₆ und R₇: gleich oder verschieden sind und jeweils für Wasserstoff, Alkyl, Cycloalkyl, Aryl oder Aralkyl,
         insbesondere für Wasserstoff, Alkyl mit 1 bis 18 Kohlenstoffatomen, das gegebenenfalls einfach oder mehrfach durch Alkoxy mit 1 bis 6 Kohlenstoffatomen, Cyano, Hydroxy oder Halogen substituiert ist, Cycloalkyl mit 3 bis 8 Kohlenstoffatomen, das gegebenenfalls ein
      fach oder mehrfach durch Alkyl oder Alkoxy mit jeweils 1 bis 6 Kohlenstoffatomen, Hydroxy oder Halogen substituiert ist, Aryl oder Aralkyl mit 1 bis 6 Kohlenstoffatomen im Alkylteil und jeweils 6 bis 10 Kohlenstoffatomen im Arylteil, welches jeweils gegebenenfalls im Arylteil einfach oder mehrfach durch Alkyl mit 1 bis 6 Kohlenstoffatomen oder Halogen substituiert ist, steht,
   oder
β) Alkoholen der Formel

   HO-R₈ (VI)

   in welcher
   - R₈: für Alkyl
   insbesondere für Alkyl mit 3 bis 18 Kohlenstoffatomen, das gegebenenfalls einfach oder mehrfach durch Alkoxy mit 1 bis 6 Kohlenstoffatomen, Cyano, Hydroxy oder Halogen substituiert ist, steht,
   umsetzt, oder
B) indem man Verbindungen der Formel (VII) 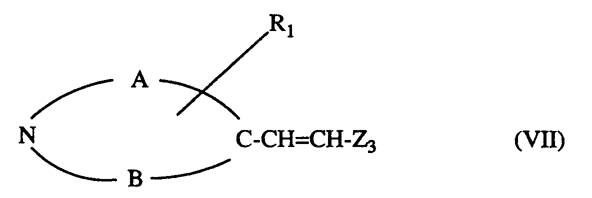 in welcher
   - A, B und R₁: die oben angegebene Bedeutung haben, und
   - Z₃: für 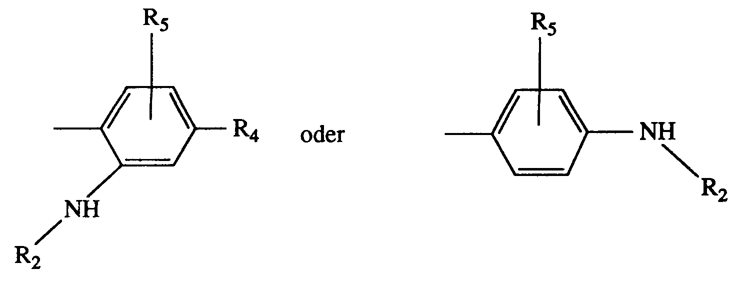 steht
   worin
   R₂, R₄ und R₅ die oben angegebene Bedeutung haben
   entweder
   a) mit Verbindungen der Formel (III) umsetzt,
      oder
   b) mit Chlorameisensäureethylester zu den entsprechenden erfindungsgemäßen, ethoxycarbonylamino-substituierten Verbindungen der Formel (I) umsetzt, und diese Verbindung gegebenenfalls durch Umsetzung mit Aminen der Formel (V) oder Alkoholen der Formel (VI) wieunter (A-b(α)) und (A-b(β)) beschrieben weiter umsetzt.

Die Verbindungen der Formel (II) sind ebenfalls neu und Gegenstand der Erfindung.

Die neuen Verbindungen der Formel (II) können hergestellt werden, indem man entweder
a) Heterocyclen der Formel 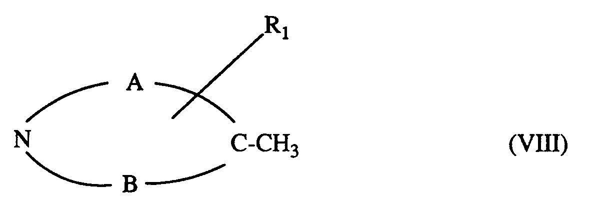 in welcher
   A, B und R₁ die oben angegebene Bedeutung haben,
   mit Aldehyden der Formel 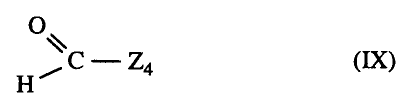 in welcher
   Z₄ für 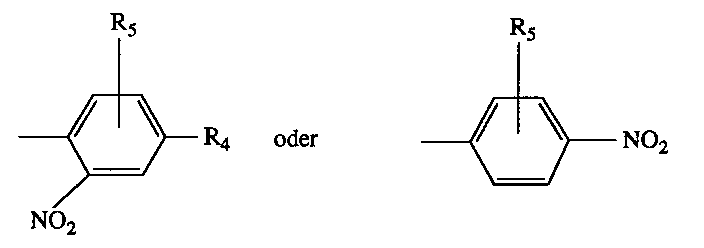
   steht,
   worin
   R₄ und R₅ die oben angegebene Bedeutung haben,
   oder mit deren Derivaten wie z.B. Anilen oder Hydrazonen, gegebenenfalls in Gegenwart eines Kondensations- oder Verdünnungsmittels wie beispielsweise Essigsäure, Essigsäureanhydrid, Sulfurylchlorid, Thionylchlorid, Oxalylchlorid oder Mischungen dieser Kondensations- oder Verdünnungsmittel bei Temperaturen zwischen beispielsweise 100°C und 140°C kondensiert, und die so erhaltenen Nitroverbindungen bei Temperaturen von beispielsweise 20°C bis 30°C mit einem Reduktionsmittel wie z.B. Wasserstoffin Gegenwart von einem Platinkatalysator an der Nitrogruppe reduziert,
   oder
b) indem man Verbindungen der Formel 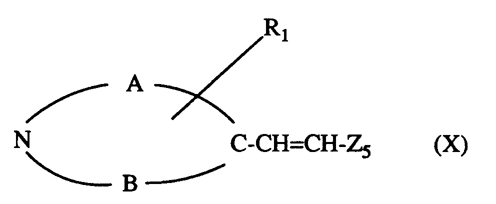 in welcher
   A, B und R₁ die oben angegebene Bedeutung haben,
   und
   Z₅ für 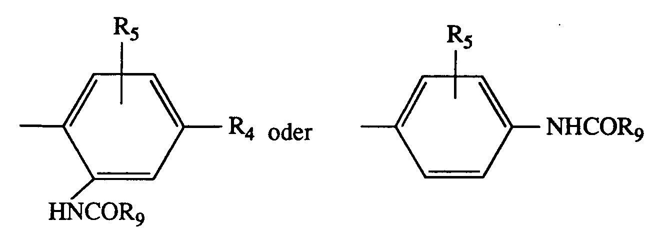 steht,
   worin
   - R₄ und R₅: die oben angegebene Bedeutung haben und
   - R₉: für CH₃ oder C₂H₅ steht,
   gegebenenfalls in Gegenwart eines Lösungsmittels wie Methanol und gegebenenfalls in Gegenwart einer Säure wie Salzsäure verseift.

Die Verbindungen der Formel (VII) sind neu und ebenfalls Gegenstand der vorliegenden Erfindung.

Die neuen Verbindungen der Formel (VII) können hergestellt werden, indem man Heterocyclen der Formel (VIII) mit Aldehyden der Formel (XI) 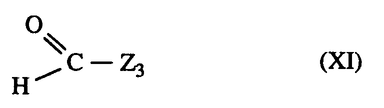
in welcher
Z₃ die oben angegebene Bedeutung hat,
gegebenenfalls in Gegenwart eines Verdünnungsmittels wie beispielsweise Essigsäure bei Temperaturen zwischen beispielsweise 100 und 140°C kondensiert.

Verbindungen der Formel (III), Amine der Formel (V), Alkohole der Formel (VI) und Heterocyclen der Formel (VIII) sind allgemein bekannte Verbindungen der organischen Chemie.

Die erfindungsgemäßen Verfahren (A) und (B) zur Herstellung der neuen Carbonylaminostyryle der Formel (I) werden vorzugsweise in Gegenwart von Verdünnungsmitteln durchgeführt. Geeignete Verdünnungsmittel sind beispielsweise Methanol, Ethanol, Pyridin.

Die Reaktionstemperaturen können bei den erfindungsgemäßen Verfahren (A) und (B) in einem größeren Bereich variiert werden. Im allgemeinen arbeitet man bei Temperaturen zwischen 80°C und 150°C, vorzugsweise bei Temperaturen zwischen 90°C und 110°C.

Die Reaktionsdurchführung und Aufarbeitung erfolgt nach allgemein üblichen Methoden.

Die bei der Herstellung der neuen Verbindungen der Formel (II) durch Kondensation von Heterocyclen (VIII) mit Aldehyden (IX) als Zwischenprodukte anfallenden Nitroverbindungen werden im allgemeinen ohne Zwischenisolierung weiter reduziert. Es ist jedoch auch möglich diese Zwischenprodukte zu isolieren, indem nach beendeter Kondensation beispielsweise auf Wasser oder Alkohol ausgetragen wird, und durch Anheben des pH-Wertes, beispielsweise mit Alkali- oder Erdalkalihydroxiden, -carbonaten, -hydrogencarbonaten, Ammoniak oder Aminen das Produkt ausgefällt wird.

Geeignete Heterocyclen (VIII) sind beispielsweise 2-Methylbenzthiazol, 2-Methylbenzoxazol, 2-Methylbenzimidazol, 2-Methylchinolin, 4-Methylchinolin, 6-Chlor-2-methylchinolin, 6-Methoxy-2-methylchinolin, 2,3,3-Trimethylindolenin, 5-Methoxy-2,3,3-trimethylindolenin, 2-Methylpyridin, 4-Methylpyridin.

Geeignete Aldehyde (XI) sind beispielsweise 2-Aminobenzaldehyd, 5-Methyl-2-aminobenzaldehyd, 4-Aminobenzaldehyd, 3-Methyl-4-aminobenzaldehyd, 2-Methoxy-4-aminobenzaldehyd, 3-Methoxy-4-aminobenzaldehyd, 2-Dodecoxy-4-aminobenzaldehyd, 2-Dodecoxy-4-amino-5-methylbenzaldehyd, 2-Chlor-4-aminobenzaldehyd, 3-Chlor-4-aminobenzaldehyd, N-Methyl-4-aminobenzaldehyd, N-Ethyl-4-aminobenzaldehyd, N-Propyl-4-amino-benzaldehyd, N-Butyl-4-aminobenzaldehyd, N-Phenyl-4-aminobenzaldehyd, 2-Methyl-4-aminobenzaldehyd, 3-Methyl-4-aminobenzaldehyd, 2-Ethyl-4-aminobenzaldehyd, 3-Ethyl-4-aminobenzaldehyd.

Geeignete Aldehyde (IX) sind beispielsweise 2-Nitrobenzaldehyd, 4-Methoxy-2-nitrobenzaldehyd, 5-Methyl-4-methoxy-2-nitrobenzaldehyd, 4-Nitrobenzaldehyd, 2-Methoxy-4-nitrobenzaldehyd, 2-Methyl-4-nitrobenzaldehyd, 3-Methyl-4-nitrobenzaldehyd, 2-Ethoxy-4-nitrobenzaldehyd, 2-Dodecoxy-4-nitrobenzaldehyd.

Geeignete Verbindungen (III) sind beispielsweise Octylsäurechlorid, Decylsäurechlorid, Dodecylsäurechlorid, Benzoesäurechlorid, 4-Methylbenzoesäurechlorid, 1-Naphtholsäurechlorid, Pyridinsäurechlorid, Hexansäurebromid, Heptansäurebromid.

Die Erfindung betrifft weiterhin die Verwendung der chromogenen Carbonylaminostyrole der Formel (I) für druckkopierfähige, thermoreaktive oder elektrochrome Aufzeichnungsmaterialien die außerdem einen sauren Farbentwickler enthalten. Für solche Aufzeichnungsmaterialien kann eine einzelne Verbindung der Formel (I) oder Gemische verschiedener Verbindungen der Formel (I) zum Einsatz gelangen, beispielsweise Gemische von Verbindungen der Formel (I), die sich nur dahingehend unterscheiden, daß sich in Molekülteil Z die 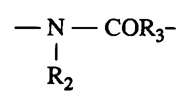
Gruppen in ortho- und para-Stellung befinden.

Als saure Entwickler sind insbesondere Tone, saure Oxide und saure Salze sowie monomere oder polymere Phenole oder Carbonsäuren zu nennen.

Die erfindungsgemäßen Farbbildner können im Gemisch mit einem oder mehreren anderen bekannten Farbbildnern, z.B. 3,3-Bis(aminophenyl)-phthaliden, 3,3-Bis(indolyl)-phthaliden, 3-Aminofluoranen, 1,3-Benzoxazinen, Spirodipyranen, Chromenoindolen, Phenoxazinen, Phenothiazinen, Carbazolylmethanen, 4,4-Diaryldihydrochinazolonen, oder anderen Triarylmethanleukofarbstoffen eingesetzt werden, insbesondereum grüne, violette, blaue oder schwarze Färbungen zu ergeben.

Ein druckempfindliches Material besteht beispielsweise aus mindestens einem Paar von Blättern, die mindestens einen Farbbildner der Formel (I), gelöst oder dispergiert in einem nichtflüchtigen organischen Lösungsmittel, und einen sauren Entwickler enthalten.

Solche Verfahren und Zubereitungen sind beispielsweise aus den US-Patentschriften 2 800 457, 2 800 458, 2 948 753, 3 096 189 und 3 193 404 und aus den deutschen Offenlegungsschriften 2 555 080 und 2 700 937 bekannt.

Um eine frühzeitige Aktivierung der in dem druckempfindlichen Aufzeichnungsmaterial vorhandenen Farbbildner zu verhindern, werden diese vorzugsweise in Mikrokapseln eingeschlossen, die sich in der Regel durch Druck zerbrechen lassen.

Als Kapselmaterial eignen sich z.B. Gelatine/Gummi arabicum, Polyamide, Polyurethane, Polysulfonamide, Polyester, Polycarbonate, Polysulfonate, Polyacrylate und Phenol- oder Harnstoff-Formaldehyd-Kondensate, wie sie z.B. in M. Gutcho, Capsule Technology and Microencapsulation, Noyes Data Corporation 1972; G. Baster, Microencapsulation, Processes and Applications, Herausgeber J.E. Vandegaar und den deutschen Offenlegungsschriften 2 237 545 und 2 119 933 beschrieben sind.

Bevorzugt werden beim erfindungsgemäßen Verfahren Mikrokapseln verwendet, deren Hüllen aus Polyadditionsprodukten aus Polyisocyanaten und Polyamiden bestehen.

Isocyanate, Amine, Lösungsmittel und ein geeignetes Herstellungsverfahren für solche Mikrokapseln sind beispielsweise in DE-OS 3 203 059 beschrieben.

Thermoreaktive Aufzeichnungssysteme umfassen z.B. wärmeempfindliche Aufzeichnungs- und Kopiermaterialien und -papiere.

Ein solches Material ist beispielsweise in der deutschen Offenlegungsschrift 2 555 080 beschrieben.

Als Entwickler eignen sich die gleichen Elektronenakzeptoren wie sie in druckempfindlichen Papieren verwendet werden, vorzugsweise phenolische Verbindungen, die z.B. in der DE-PS 1 251 348 beschrieben sind, sowie Borsäure und organische, vorzugsweise aliphatische Dicarbonsäuren.

Ein weiteres geeignetes thermoreaktives Entwicklungssystem ist das in DE-OS 3 337 296 beschriebene, bei dem sauer modifizierte Polymerisate vorzugsweise des Acrylnitrils als Entwickler wirken.

Die erfindungsgemäßen Verbindungen sind den konventionellen Gelb-Farbentwicklerverbindungen in der Summe der Eigenschaften (Intensität, Migration, Rohpapierfärbungen und Lichtechtheit) überlegen.

### Herstellungsbeispiele

### Beispiel 1

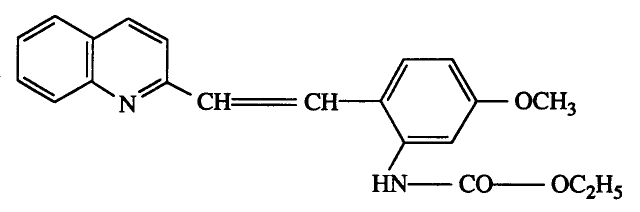

100 g 4-Methoxy-2-nitrobenzaldehyd werden in 400 ml Essigsäure suspendiert, mit 82 g Chinaldin versetzt und 10 Stunden unter Rückfluß gekocht. Das Gemisch wird auf 6 l Eiswasser ausgetragen, filtriert und in 1.5 l Methanol gelöst. Die Lösung wird mit einem Pt-Katalysator reduziert. Die Lösung wird filtriert und eingeengt. Die Substanz wird in 600 ml Pyridin gelöst und mit 43 g Chlorameisensäureethylester versetzt. Das Gemisch wird 12 Stunden gerührt und auf 6 l Wasser ausgetragen. Die Substanz wird in Toluol aufgenommen und eingeengt. Ausbeute: 60 g, λₘₐₓ (Essigsäure) = 414 nm. Auf Clay entwickelt das Produkt mit einer gelben Farbe.

### Beispiel 2

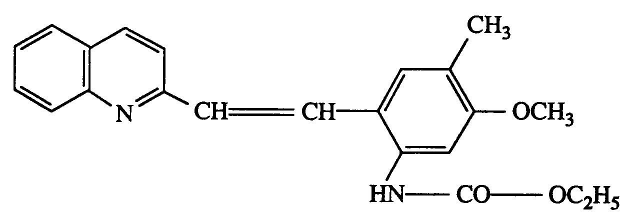

112 g 5-Methyl-4-methoxy-2-ethoxycarbonylaminobenzaldehyd werden in 300 ml Eisessig vorgelegt, mit 71.5 g Chinaldin versetzt und 3 Stunden unter Rückfluß erwärmt. Das Gemisch wird auf 5 l Eiswasser ausgetragen und mit 50 %iger Natronlauge alkalisch gestellt. Der Niederschlag wird abfiltriert, neutral gewaschen und im Vakuum getrocknet. Ausbeute: 73 g; λₘₐₓ =416 nm (Essigsäure).

### Beispiel 3

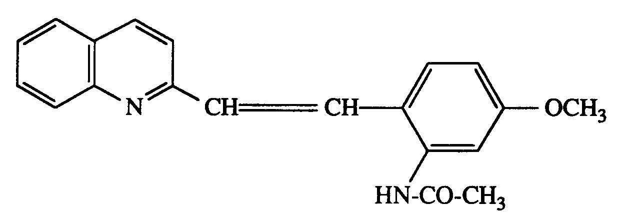

212 g 4-Methoxy-2-aminoanil werden in 400 ml Essigsäureanhydrid suspendiert, mit 72 g Chinaldin versetzt und 8 Stunden unter Rückfluß gekocht. Das Gemisch wird auf 5 l Eiswasser ausgetragen, filtriert und das Filtrat mit 50 %iger Natronlauge alkalisch gestellt. Der Rückstand wird abgesaugt. Das Produkt kristallisiert mit einer Ausbeute von 112g; λₘₐₓ =403 nm (Essigsäure).

Analog zu den Beispielen 1, 2 und 3 und gegebenenfalls durch anschließende weitere Umsetzung mit Alkoholen (VI) oder Aminen (V) wurden die in Tabelle 1 aufgeführten Verbindungen der allgemeinen Formel 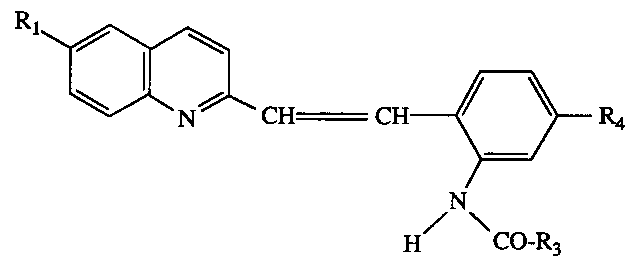
erhalten.

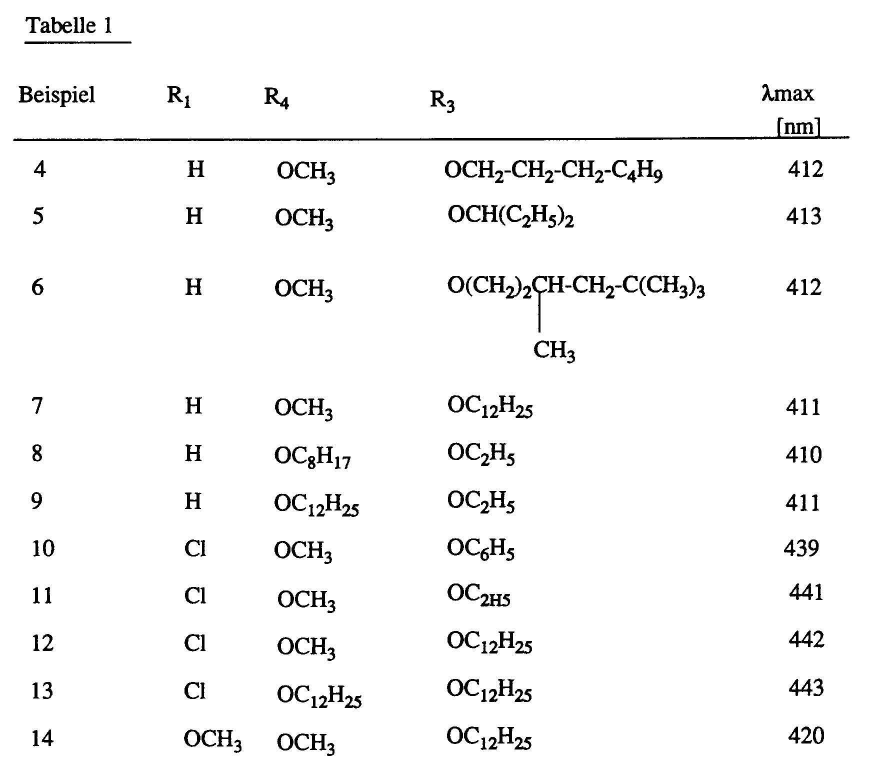

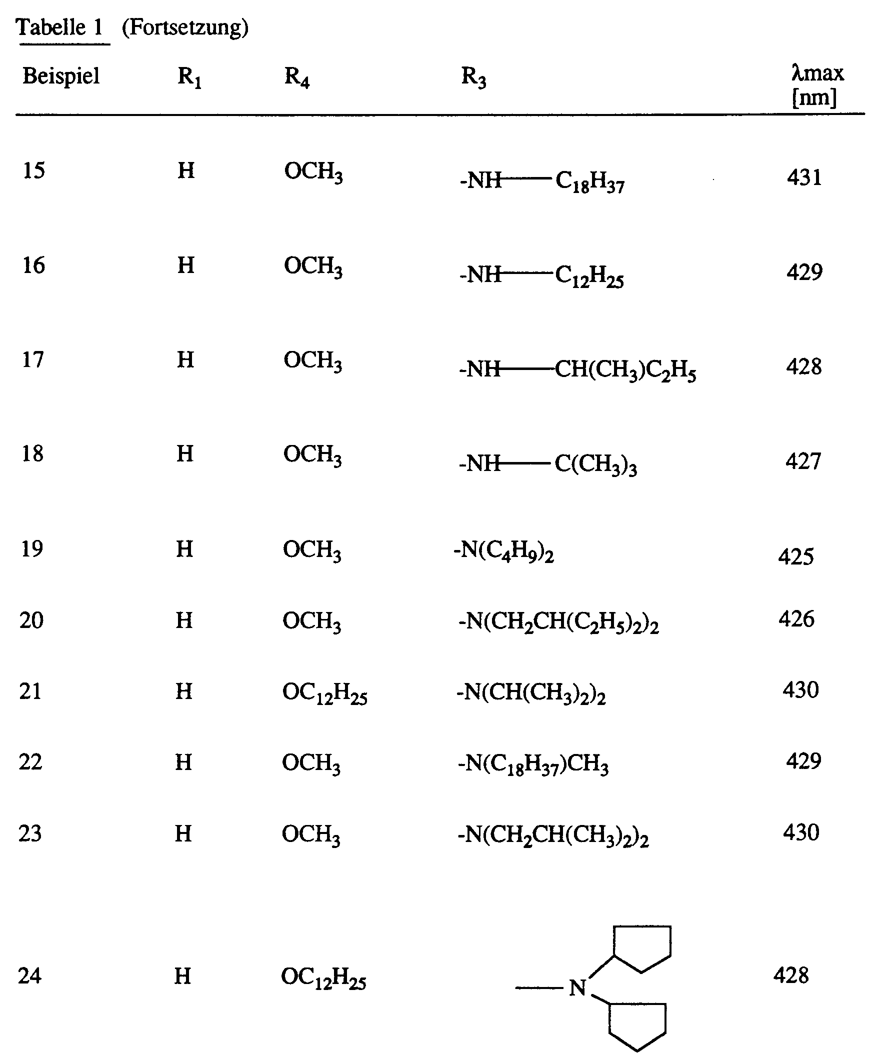

### Beispiel 25

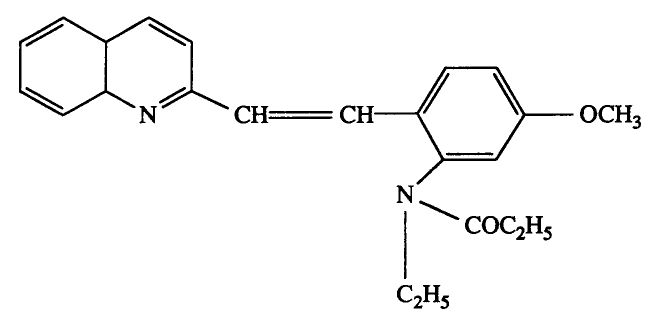

72 g Chinaldin und 75 g 4-Methoxy-2-(N-ethylamino)-benzaldehyd werden in 300 ml Essigsäure 8 Stunden auf 100°C erhitzt. Das Gemisch wird auf 3 l Eiswasser ausgetragen und bei Raumtemperatur mit 50 %-iger Natronlauge alkalisch gestellt. Der Niederschlag wird abgesaugt und getrocknet. Dann wird er in 300 ml Pyridin vorgelegt, mit 65 g Chlorameisensäureethylester versetzt und 2 Stunden auf 80°C erhitzt. Das Gemisch wird auf 600 ml Wasser ausgetragen und abgesaugt. Das Produkt kristallisiert mit einer Ausbeute von 75 g, λₘₐₓ=396 nm (Essigsäure).

Analog zu Herstellungsbeispiel 25 und gegebenenfalls durch anschließende weitere Umsetzung mit Alkoholen (VI) oder Aminen (V) wurden die in Tabelle 2 aufgeführten Verbindungen der allgemeinen Formel 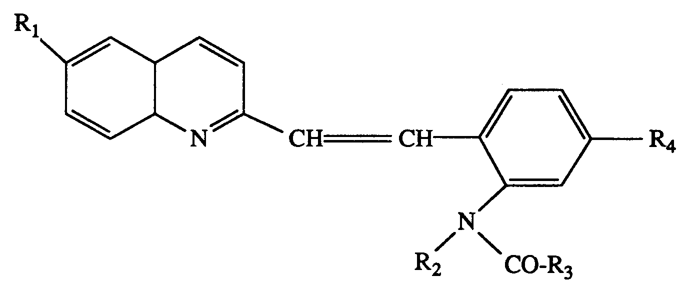
hergestellt.

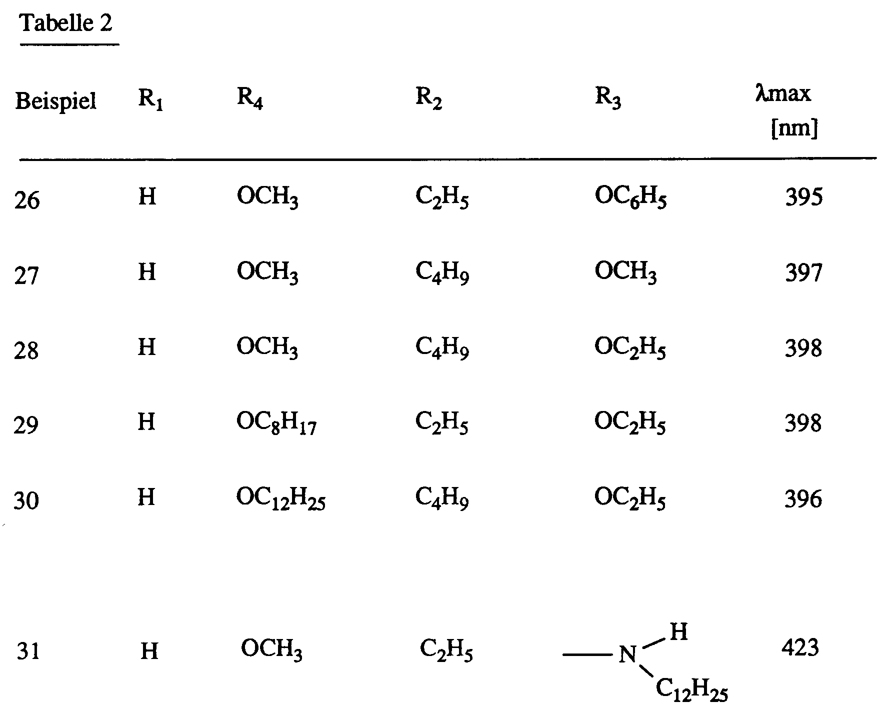

Analog zu den Herstellungsbeispielen 1,2 und 3 und gegebenenfalls durch anschließende weitere Umsetzung mit Alkoholen (VI) oder Aminen (V) wurden die in Tabelle 3 aufgeführten Verbindungen der allgemeinen Formel 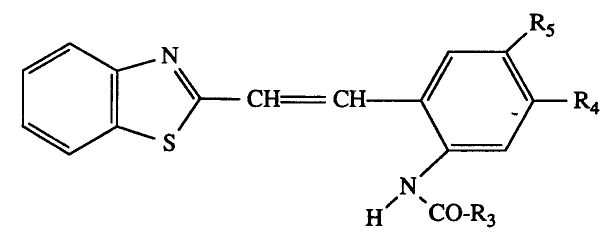
erhalten.

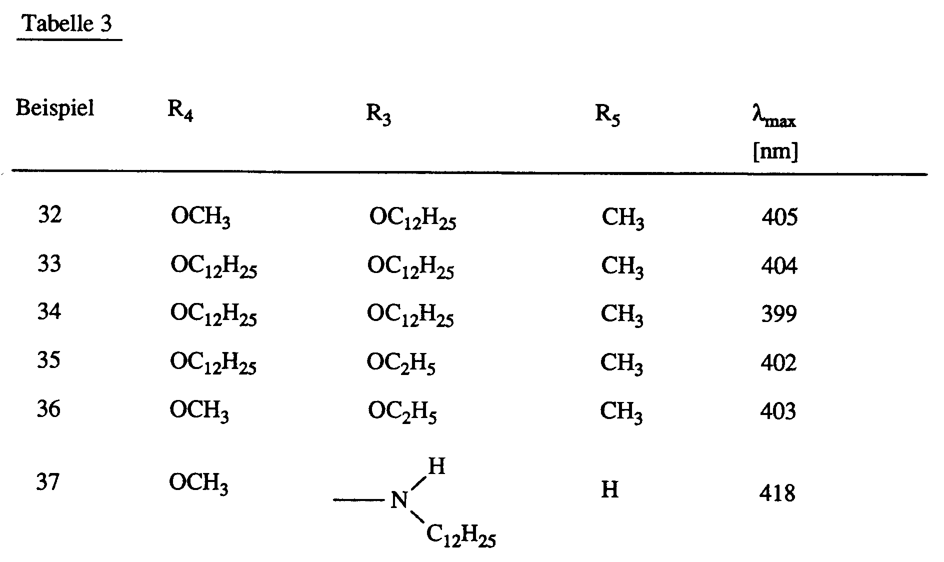

### Beispiel 38

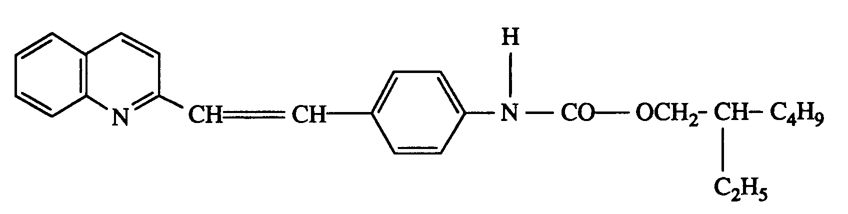

212 g 4-Aminoanil werden in 400 ml Essigsäureanhydrid suspendiert, mit 72 g Chinaldin versetzt und 8 Stunden unter Rückfluß gekocht. Das Gemisch wird auf 5 l Eiswasser ausgetragen, filtriert und das Filtrat mit 50% Natronlauge alkalisch gestellt. Der Rückstand wird abgesaugt und aus Methanol umkristallisiert. Das Produkt wird in 450 ml Methanol unter Rückfluß erwärmt, mit 190 g 10% Salzsäure versetzt 24 Stunden lang refluxiert. Das Gemisch wird auf 1.5 l Wasser ausgetragen, mit 50%-iger Natronlauge alkalisch gestellt und abgesaugt. Das getrocknete Produkt wird in 300 ml Pryridin vorgelegt, mit 34 g Chlorameisensäureethylester versetzt und über Nacht bei Raumtemperatur gerührt. Das Gemisch wird auf 300 ml Wasser ausgetragen und abgesaugt. Das getrocknete Produkt wird in 117 g 2-Ethyl-1-hexanol auf 180°C erhitzt und dabei wird Ethanol abdestilliert. Nach vollständiger Umsetzung wird das Gemisch im Vakuum eingeengt. Das Produkt kristallisiert mit einer Ausbeute von 63 g aus. λmax=410 nm (Essigsäure). Auf Clay entwickelt das Produkt mit einer gelben Farbe.

### Beispiel 39

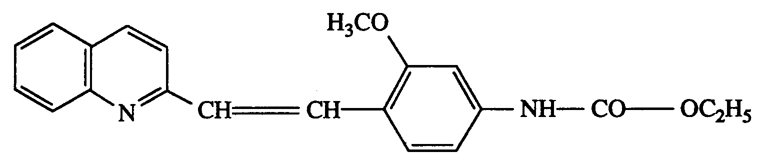

112 g 2-Methoxy-4-ethoxycarbonylaminobenzaldehyd werden in 300 ml Eisessig vorgelegt, mit 71:5 g Chinaldin versetzt und 3 Stunden unter Rückfluß erwärmt. Das Gemisch wird auf 5 l Eiswasser ausgetragen und mit 50% Natronlauge alkalisch gestellt. Der Niederschlag wird abfiltriert, neutral gewaschen und im Vakuum getrocknet. Ausbeute: 73 g; λmax=414 nm (Essigsäure).

### Beispiel 40

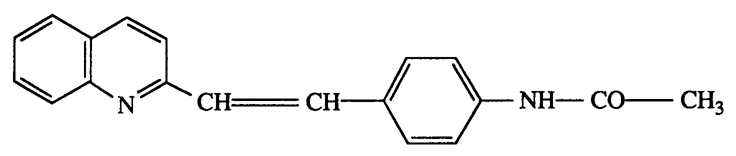

212 g 4-Aminoanil werden in 400 ml Essigsäureanhydrid suspendiert, mit 72 g Chinaldin versetzt und 8 Stunden unter Rückfluß gekocht. Das Gemisch wird auf 5 l Eiswasser ausgetragen, filtriert und das Filtrat mit 50 %-iger Natronlauge alkalisch gestellt. Der Rückstand wird abgesaugt. Das Produkt kristallisiert mit einer Ausbeutevon 96 g; λₘₐₓ(Eisessig): 401 nm.

Analog zu den Herstellungsbeispielen 38, 89 und 40 und gegebenenfalls durch weitere Derivatisierung mittels Aminen (V) oder Alkoholen (VI) wurden die in Tabelle 4 aufgeführten Verbindungen der allgemeinen Formel 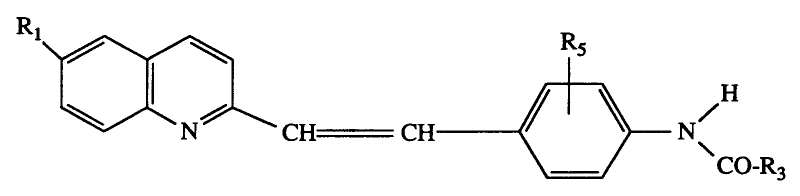
hergestellt.

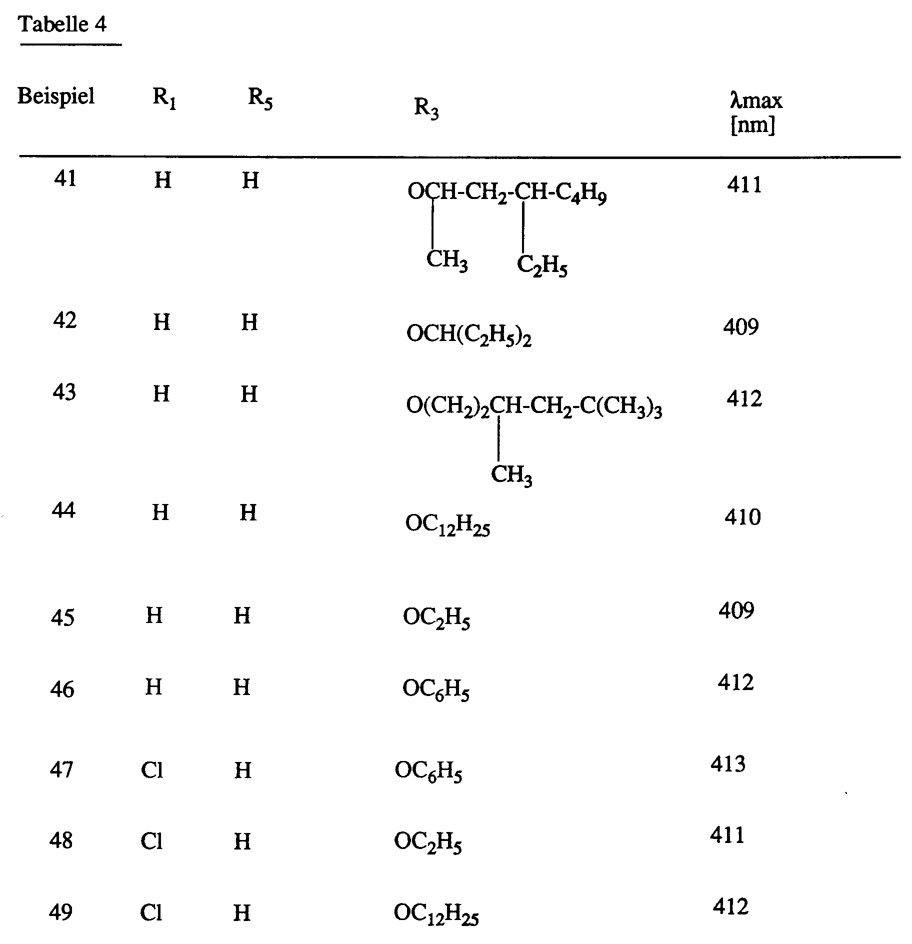

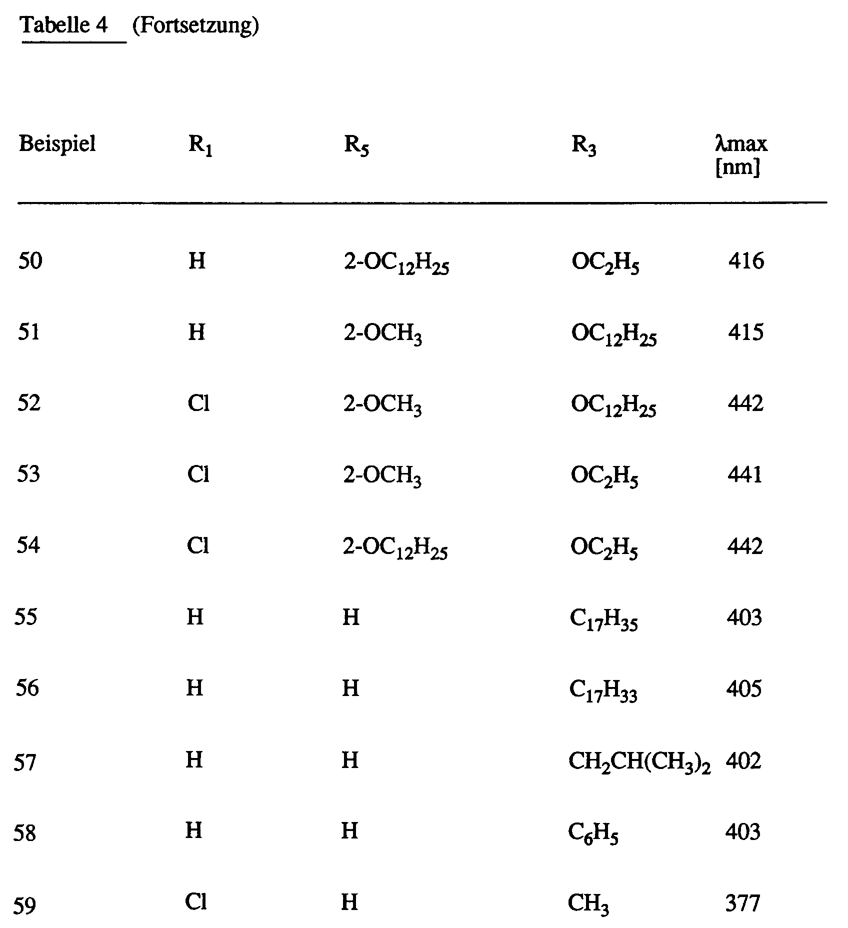

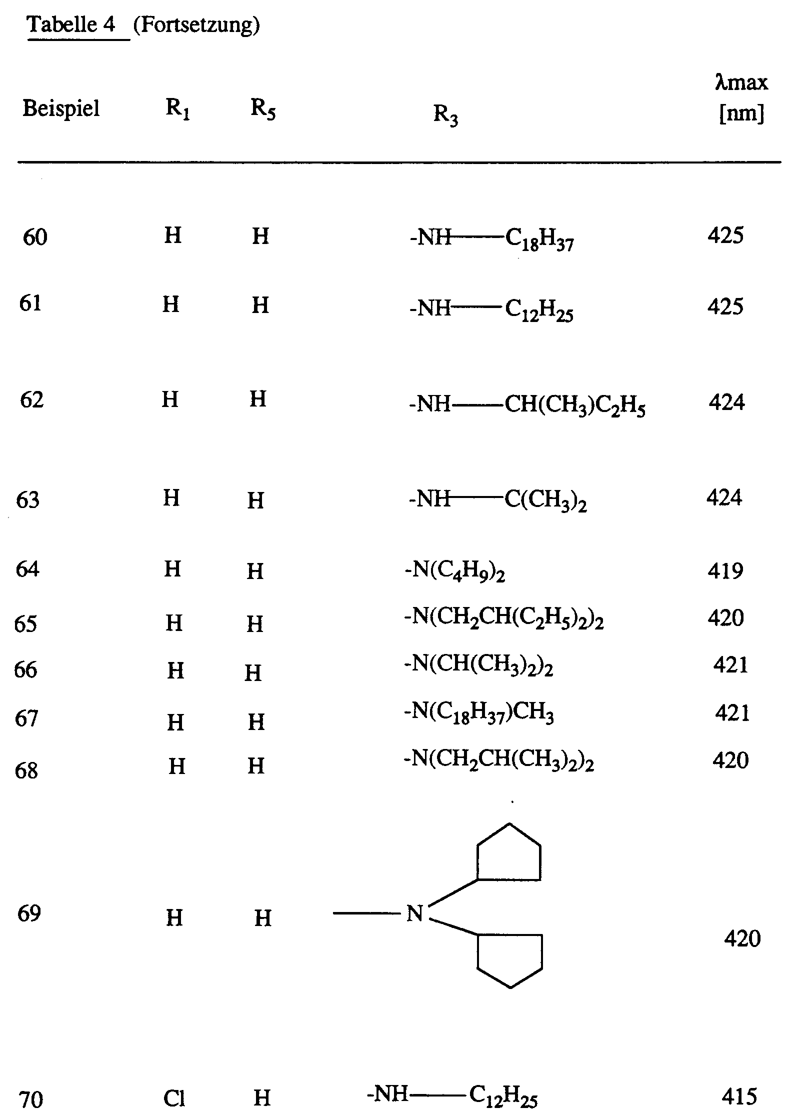

Analog zu den Beispielen 38, 39, 40 und gegebenenfalls durch anschließende weitere Derivatisierung mit Aminen (V) oder Alkoholen (VI) wurden die in Tabelle 5 aufgeführten Verbindungen der allgemeinen Formel 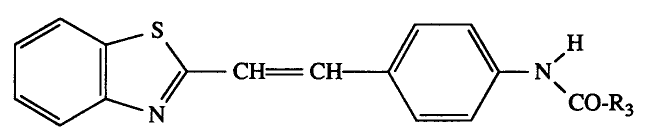
hergestellt.

**Tabelle 5**

| Beispiel | R₃ | λmax [nm] |
|---|---|---|
| 71 | OC₂H₅ | 358 |
| 72 | OC₆H₅ | 360 |
| 73 | OC₁₂H₂₅ | 359 |
| 74 | -NH-C₁₂H₂₅ | 359 |

### Beispiel 75

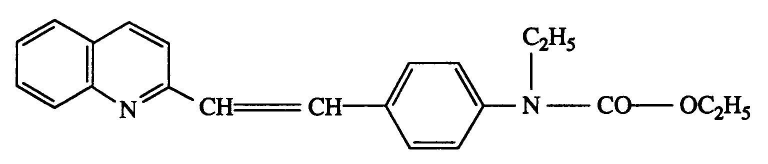

71,5 g Chinaldin und 75 g 4-(N-Ethylamino)-benzyldehyd werden in 300 ml Essigsäure 7 Stunden auf 100°C erhitzt. Das Gemisch wird auf 3 l Eiswasser ausgetragen und bei Raumtemperatur mit 50 %-iger Natronlauge alkalisch gestellt. Der Niederschlag wird abgesaugt und getrocknet. Das Produkt wird in 300 ml Pyridin vorgelegt, mit 65 g Chlorameisensäureethylester versetzt und 2 Stunden auf 80°C erhitzt. Das Gemisch wird auf 600 ml Wasser ausgetragen und abgesaugt. Das Produkt kristallisiert mit einer Ausbeute von 82 g; λₘₐₓ=383 nm (Essigsäure).

Analog zu Beispiel 75 und gegebenenfalls durch weitere Umsetzung mit Aminen (V) oder Alkoholen (VI) wurden die in Tabelle 6 aufgeführten Verbindungen der allgemeinen Formel 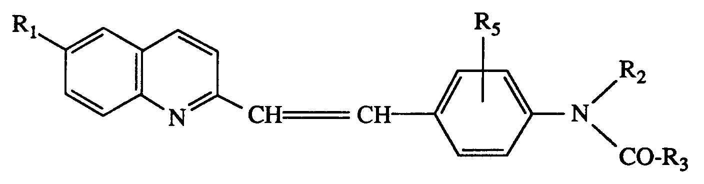
hergestellt.

**Tabelle 6**

| Beispiel | R₁ | R₅ | R₂ | R₃ | λmax [nm] |
|---|---|---|---|---|---|
| 76 | H | H | C₂H₅ | OC₆H₅ | 389 |
| 77 | H | H | C₂H₅ | OC₂H₅ | 384 |
| 78 | H | 2-CH₃ | C₄H₉ | OC₂H₅ | 392 |
| 79 | H | 3-CH₃ | C₂H₅ | OC₂H₅ | 390 |
| 80 | H | H | C₄H₉ | OC₂H₅ | 391 |

## Patentansprüche

1. Carbonylaminostyryle der Formel 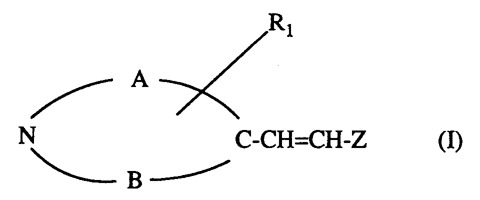 in welcher
A für die restlichen Glieder eines gegebenenfalls substituierten heterocyclischen Ringsystems, an weiches gegebenenfalls ein weiterer Carbocyclus anelliert ist, steht,
B für eine Doppelbindung oder die Gruppierung =CH-CH= steht,
R₁ für Wasserstoff, Alkyl, Alkenyl, Cycloalkyl, Alkoxy oder Halogen steht, und
Z für 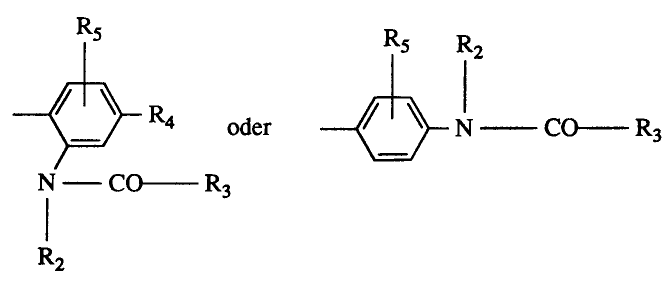 steht,
worin
R₂ für Wasserstoff, Alkyl, Alkenyl, Cycloalkyl, Aralkyl, Aryl oder Hetaryl steht,
R₃ für Wasserstoff, Alkyl, Alkenyl, Cycloalkyl, Aralkyl, Aryl, Alkoxy, Dialkylamino, Alkylamino, Cycloalkylamino, Arylamino, Aralkylamino oder Hetaryl steht, oder
R₂ und R₃ gemeinsam mit dem Stickstoffatom und dem Kohlenstoffatom, an welches sie gebunden sind einen gegebenenfalls substituierten 4- bis 8-gliedrigen Ring bilden, oder
R₂ und R₅, für den Fall, daß R₅ in ortho-Stellung zum Stickstoff steht, gemeinsam mit dem Stickstoffatom und dem Kohlenstoffatom an welches sie gebunden sind einen gegebenenfalls substituierten 5- bis 7-gliedrigen Ring bilden, und
R₄ und R₅ unabhängig voneinander für Wasserstoff, Alkyl, Alkenyl, Cycloalkyl, Alkoxy oder Halogen stehen.

2. Carbonylaminostyryle des Anspruchs 1, worin
A für die restlichen Glieder eines 5- bis 7-gliedrigen, partiell gesättigten oder ungesättigten heterocyclischen Ringsystems steht, welches gegebenenfalls 1 oder 2 weitere Heteroatome aus der Reihe Sauerstoff, Schwefel, Stickstoff enthält, und an welches gegebenenfalls ein gesättigter oder ungesättigter 5- bis 7-gliedriger Carbocyclus anelliert ist,
B für eine Doppelbindung oder die Gruppierung =CH-CH= steht,
R₁ für Wasserstoff, Alkyl mit 1 bis 8 Kohlenstoffatomen, welches gegebenenfalls einfach oder mehrfach durch Alkoxy mit 1 bis 6 Kohlenstoffatomen, Cyano, Hydroxy oder Halogen substituiert ist, Cycloalkyl mit 3 bis 8 Kohlenstoffatomen, Alkenyl mit 2 bis 6 Kohlenstoffatomen, Alkoxy mit 1 bis 6 Kohlenstoffatomen oder Halogen steht, und
in Z
R₂ für Wasserstoff, Alkyl mit 1 bis 8 Kohlenstoffatomen, welches gegebenenfalls einfach oder mehrfach durch Alkoxy mit 1 bis 6 Kohlenstoffatomen, Hydroxy, Cyano oder Halogen substituiert ist, Alkenyl mit 2 bis 6 Kohlenstoffatomen, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Hydroxy oder Halogen substituiert ist, Aryl oder Aralkyl mit 1 bis 6 Kohlenstoffatomen im Alkylteil und jeweils 6 bis 10 Kohlenstoffatomen im Arylteil, welches jeweils gegebenenfalls im Arylteil einfach oder mehrfach durch Alkyl mit 1 bis 6 Kohlenstoffatomen oder Halogen substituiert ist oder für einen gesättigten oder ungesättigten 5- bis 7-gliedrigen heterocyclischen Ring, der 1 bis 4 Heteroatome aus der Reihe Sauerstoff, Schwefel, Stickstoff enthält und gegebenenfalls einfach oder mehrfach durch Alkyl mit 1 bis 6 Kohlenstoffatomen substituiert ist, und an welchen gegebenenfalls ein weiterer gesättigter oder ungesättigter 5- bis 6-gliedriger Carbocyclus anelliert ist, steht,
R₃ für Wasserstoff, Alkyl oder Alkoxy mit jeweils 1 bis 18 Kohlenstoffatomen, das jeweils gegebenenfalls einfach oder mehrfach durch Alkoxy mit 1 bis 6 Kohlenstoffatomen, Cyano, Hydroxy oder Halogen substituiert ist, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Hydroxy oder Halogen substituiert ist, Aryl oder Aralkyl mit 1 bis 6 Kohlenstoffatomen im Alkylteil und jeweils 6 bis 10 Kohlenstoffatomen im Arylteil, welches jeweils gegebenenfalls im Arylteil einfach oder mehrfach durch Alkyl mit 1 bis 6 Kohlenstoffatomen oder Halogen substituiert ist, Alkenyl mit 2 bis 6 Kohlenstoffatomen, Dialkylamino oder Alkylamino mit jeweils 1 bis 18 Kohlenstoffatomen in den jeweiligen Alkylteilen, Cycloalkylamino mit 3 bis 8 Kohlenstoffatomen, Arylamino oder Aralkylamino mit 1 bis 6 Kohlenstoffatomen im Alkylteil und jeweils 6 bis 10 Kohlenstoffatomen in den jeweiligen Arylteilen, welches jeweils gegebenenfalls einfach oder mehrfach im Arylteil durch Alkyl mit 1 bis 6 Kohlenstoffatomen oder Halogen substituiert ist, oder für einen gesättigten oder ungesättigten 5- bis 7-gliedrigen heterocyclischen Ring, der 1 bis 4 Heteroatome wie Sauerstoff, Schwefel oder Stickstoff enthält und gegebenenfalls einfach oder mehrfach durch Alkyl mit 1 bis 6 Kohlenstoffatomen substituiert ist, steht, oder
R₂ und R₃ gemeinsam mit dem Stickstoffatom bzw. dem Kohlenstoffatom an welches sie gebunden sind einen gesättigten oder ungesättigten 4- bis 8-gliedrigen Ring bilden, der gegebenenfalls einfach oder mehrfach durch Alkyl mit 1 bis 6 Kohlenstoffatomen und/oder Oxo substituiert ist, oder
R₂ und R₅, für den Fall, daß R₅ in ortho-Stellung zum Stickstoff steht, gemeinsam mit dem Stickstoffatom bzw. dem Kohlenstoffatom an welches sie gebunden sind einen gesättigten oder ungesättigten 5- bis 7-gliedrigen Ring bilden, der gegebenenfalls einfach oder mehrfach durch Alkyl mit 1 bis 6 Kohlenstoffatomen substituiert ist, und
R₄ und R₅ unabhängig voneinander für Wasserstoff, Alkyl mit 1 bis 18 Kohlenstoffatomen, das gegebenenfalls einfach oder mehrfach durch Alkoxy mit 1 bis 6 Kohlenstoffatomen, Cyano, Hydroxy oder Halogen substituiert ist, Alkenyl mit 2 bis 6 Kohlenstoffatomen, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Cyano oder Halogen substituiert ist, Alkoxy mit 1 bis 18 Kohlenstoffatomen oder Halogen stehen.

3. Carbonylaminostyryle des Anspruchs 1, worin
A für die restlichen Glieder eines 5- bis 6-gliedrigen, partiell gesättigten oder ungesättigten heterocyclischen Ringsystems steht, welches gegebenenfalls 1 oder 2 weitere Heteroatome aus der Reihe Sauerstoff, Schwefel,Stickstoff enthält, und an welches gegebenenfalls ein ungesättigter 6-gliedriger Carbocyclus anelliert ist,
B für eine Doppelbindung oder die Gruppierung =CH-CH= steht,
R₁ für Wasserstoff, Alkyl mit 1 bis 6 Kohlenstoffatomen, das gegebenenfalls 1- bis 3-fach durch Alkoxy mit 1 bis 4 Kohlenstoffatomen, Cyano, Hydroxy, Fluor, Chlor oder Brom substituiert ist, Cycloalkyl mit 3 bis 7 Kohlenstoffatomen, Alkenyl mit 2 bis 4 Kohlenstoffatomen, Alkoxy mit 1 bis 4 Kohlenstoffatomen, Fluor, Chlor oder Brom steht, und
in Z
R₂ für Wasserstoff, Alkyl mit 1 bis 6 Kohlenstoffatomen, das gegebenenfalls 1- bis 3-fach durch Alkoxy mit 1 bis 4 Kohlenstoffatomen, Hydroxy, Cyano, Fluor, Chlor oder Brom substituiert ist, Alkenyl mit 2 bis 4 Kohlenstoffatomen, Cycloalkyl mit 3 bis 7 Kohlenstoffatomen, welches gegebenenfalls 1- bis 3-fach durch Alkoxy oder Alkyl mit jeweils 1 bis 4 Kohlenstoffatomen, Hydroxy, Fluor, Chlor oder Brom substituiert ist, Phenyl, Naphthyl, Benzyl oder Phenethyl, welches jeweils gegebenenfalls 1 bis 3-fach im Phenyl- bzw. Naphthylteil durch Alkyl mit 1 bis 4 Kohlenstoffatomen, Fluor, Chlor oder Brom substituiert ist, oder für einen gesättigten oder ungesättigten 5- oder 6-gliedrigen heterocyclischen Ring, der 1 bis 3 Heteroatome aus der Reihe Sauerstoff, Schwefel, Stickstoff enthält, und an welchen gegebenenfalls ein weiterer ungesättigter 5- bis 6-gliedriger Carbocyclus anelliert ist, steht,
R₃ für Wasserstoff, Alkyl oder Alkoxy mit jeweils 1 bis 18 Kohlenstoffatomen, das jeweils gegebenenfalls 1- bis 3-fach durch Alkoxy mit 1 bis 4 Kohlenstoffatomen, Cyano, Hydroxy, Fluor, Chlor oder Brom substituiert ist, Cycloalkyl mit 3 bis 7 Kohlenstoffatomen, das gegebenenfalls 1- bis 3-fach durch Alkyl oder Alkoxy mit jeweils 1 bis 4 Kohlenstoffatomen, Hydroxy, Fluor, Chlor oder Brom substituiert ist, Phenyl, Naphthyl, Benzyl oder Phenethyl welches jeweils gegebenenfalls im Phenyl- bzw. Naphthylteil 1- bis 3-fach durch Alkyl mit 1 bis 4 Kohlenstoffatomen, Fluor, Chlor oder Brom substituiertist, Alkenyl mit 2 bis 4 Kohlenstoffatomen, Dialkylamino oder Alkylamino mit jeweils 1 bis 18 Kohlenstoffatomen in den jeweiligen Alkylteilen, Cycloalkylamino mit 3 bis 7 Kohlenstoffatomen, Phenylamino, Naphthylamino, Benzylamino oder Phenethylamino welches jeweils gegebenenfalls 1- bis 3-fach im Phenyl- bzw. Naphthylteil durch Alkyl mit 1 bis 4 Kohlenstoffatomen, Fluor, Chlor oder Brom substituiert ist, oder für einen gesättigten oder ungesättigten 5- bis 7-gliedrigen heterocyclischen Ring, der 1 bis 3 Heteroatome aus der Reihe Sauerstoff, Schwefel, Stickstoff enthält, und 1- bis 4-fach durch Alkyl mit 1 bis 4 Kohlenstoffatomen substituiert ist, steht, oder
R₂ oder R₃ gemeinsam mit dem Stickstoffatom bzw. dem Kohlenstoffatom an welches sie gebunden sind einen gesättigten oder ungesättigten 4-bis 8-gliedrigen Ring bilden, der gegebenenfalls 1- bis 6-fach durch Alkyl mit 1 bis 4 Kohlenstoffatomen und/oder Oxo substituiert ist, oder
R₂ und R₅, für den Fall, daß R₅ in ortho-Stellung zum Stickstoff steht, gemeinsam mit dem Stickstoffatom bzw. dem Kohlenstoffatom an welches sie gebunden sind einen gesättigten oder ungesättigten 5- bis 7-gliedrigen Ring bilden, der gegebenenfalls 1- bis 3-fach durch Alkyl mit 1 bis 4 Kohlenstoffatomen substituiert ist, und
R₄ und R₅ unabhängig voneinander für Wasserstoff, Alkyl mit 1 bis 16 Kohlenstoffatomen, das gegebenenfalls 1- bis 3-fach durch Alkoxy mit 1 bis 4 Kohlenstoffatomen, Cyano, Hydroxy oder Halogen substituiert ist, Alkenyl mit 2 bis 4 Kohlenstoffatomen, Cycloalkyl mit 3 bis 7 Kohlenstoffatomen, das gegebenenfalls 1- bis 3-fach durch Alkyl oder Alkoxy mit jeweils 1 bis 4 Kohlenstoffatomen, Cyano oder Halogen substituiert ist, Alkoxy mit 1 bis 16 Kohlenstoffatomen, Fluor, Chlor oder Brom stehen.

4. Carbonylaminostyryle des Anspruchs 1, worin 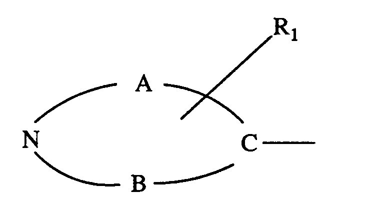 für 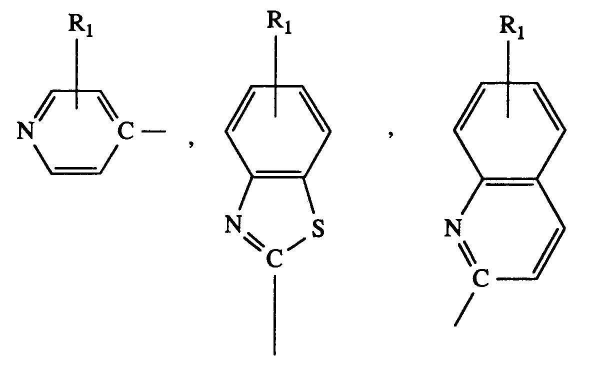 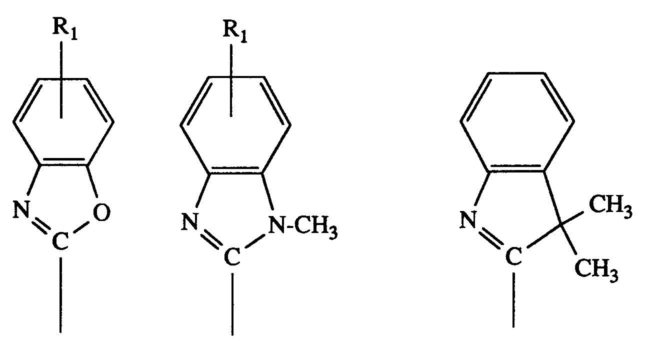
R₁ für Wasserstoff, Alkyl mit 1 bis 4 Kohlenstoffatomen, das gegebenenfalls durch Methoxy, Ethoxy, Cyano, Hydroxy oder Chlor substituiert ist, Cyclopentyl, Cyclohexyl, Vinyl, Allyl, Methoxy, Ethoxy, n-Propoxy oder Chlor steht, und
in Z
R₂ für Wasserstoff, Alkyl mit 1 bis 4 Kohlenstoffatomen, das gegebenenfalls durch Methoxy, Ethoxy, Cyano, Hydroxy oder Chlor substituiert ist, Cyclopentyl, Cyclohexyl, Vinyl, Allyl; für Phenyl oder Benzyl welches jeweils gegebenenfalls im Phenylteil durch Methyl, Methoxy, Cyano oder Chlor substituiert ist, oder für Pyridyl, Pyrimidyl, Benzimidazolyl, Benzoxazolyl, Benzthiazolyl oder Chinolyl steht,
R₃ für Wasserstoff, Alkyl oder Alkoxy mit jeweils 1 bis 18 Kohlenstoffatomen, das jeweils gegebenenfalls durch Methoxy, Ethoxy, Cyano, Hydroxy oder Chlor substituiert ist; für Cyclopentyl, Cyclohexyl, Phenyl, Benzyl oder Naphthyl, welches jeweils gegebenenfalls durch Methyl, Methoxy, Cyano oder Chlor substituiert ist; für Vinyl, Allyl; Dialkylamino oder Alkylamino mit jeweils 1 bis 18 Kohlenstoffatomen in den jeweiligen Alkylteilen, Cyclopentylamino, Cyclohexylamino, Phenylamino, oder für Pyridyl, Pyrimidyl, Thienyl, Furyl, Pyrrolyl, Pyrazinyl, 1-Pyrrolidyl oder 1-Piperidyl steht, wobei die heterocyclischen Reste jeweils gegebenenfalls durch 1 bis 4 Methylgruppen substituiert sind, oder
R₂ und R₃ gemeinsam mit dem Stickstoffatom, bzw. dem Kohlenstoffatom an welches sie gebunden sind, einen 5- oder 6-gliedrigen gesättigten oder ungesättigten Ring bilden, der gegebenenfalls 1- bis 6-fach durch Methyl und/oder Oxo substituiert ist, oder
R₂ und R₅, für den Fall, daß R₅ in ortho-Stellung zum Stickstoff steht, gemeinsam mit dem Stickstoffatom bzw. dem Kohlenstoffatom an welches sie gebunden sind einen gesättigten oder ungesättigten 5- bis 6-gliedrigen Ring bilden, der gegebenenfalls 1- bis 3-fach durch Methyl substituiert ist,
R₄ für Wasserstoff, Alkyl mit 1 bis 14 Kohlenstoffatomen, das gegebenenfalls durch Methoxy, Ethoxy, Cyano, Hydroxy oder Chlor substituiert ist, Cyclopentyl, Cyclohexyl, Vinyl, Allyl, Alkoxy mit 1 bis 14 Kohlenstoffatomen oder Chlor steht, und
R₅ für Wasserstoff, Alkyl oder Alkoxy mit jeweils 1 bis 14 Kohlenstoffatomen steht.

5. Carbonylaminostyryle des Anspruchs 1, worin 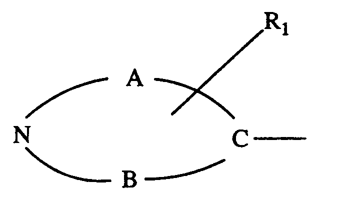 für 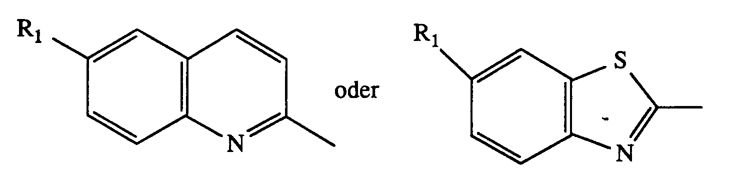 steht,
R₁ für Wasserstoff, Methyl, Ethyl, Hydroxymethyl, Chlormethyl, Methoxymethyl, Chlorethyl, Cyanomethyl, Hydroxyethyl, Methoxy, Ethoxy oder Chlor steht,
in Z
R₂ für Wasserstoff, Methyl, Ethyl, n-Propyl, i-Propyl, n-Butyl, s-Butyl, i-Butyl, t-Butyl, Hydroxymethyl oder Hydroxyethyl steht,
R₃ für Wasserstoff, Methyl, Ethyl, Propyl, Hexyl, Heptyl, Octyl, 2-Methylpropyl, Nonyl, Decyl, Dodecyl, Hexadecyl, Octadecyl, Cyclopentyl, Cyclohexyl, Benzyl, Phenyl, Methoxy, Ethoxy, Propoxy, Butoxy, Pentoxy, Hexoxy, Heptoxy, Octoxy, 2-Ethylhexoxy, 1,3-Dimethylbutoxy, 1-Ethylpropoxy, 3,5,5-Trimethylhexoxy, Dodecyloxy, Hexadecyloxy, Octadecyloxy, Dimethylamino, Diethylamino, Dipropylamino, Dibutylamino, Diisopropylamino, Di(2-ethylbutyl)amino, Di(2-methylpropyl)amino, 1-Methylpropanamino, 1,1-Dimethylethanamino, Octylamino, Decylamino, Dodecylamino, Hexadecylamino, Octadecylamino, Cyclobutylamino, Cyclopentylamino, Cyclohexylamino steht,
R₄ für Wasserstoff, Methyl, Ethyl, Propyl, Butyl, Hexyl, Methoxy, Ethoxy, Propoxy, Butoxy, Hexoxy, Octoxy, Decyloxy, Dodecyloxy oder Chlor steht, und
R₅ für Wasserstoff, Methyl, Ethyl, Propyl, Butyl, Hexyl, Methoxy, Ethoxy, Propoxy, Butoxy, Hexoxy, Octoxy, Decyloxy, Dodecyloxy steht.

6. Carbonylaminostyryle des Anspruchs 1 der Formel 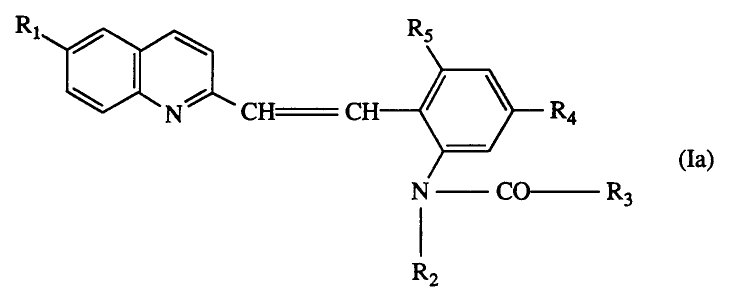 in welcher
R₁ für Wasserstoff, Methyl, Ethyl, Methoxy, Ethoxy oder Chlor steht,
R₂ für Wasserstoff, Methyl, Ethyl, n-Propyl, i-Propyl, n-Butyl, s-Butyl, i-Butyl oder t-Butyl steht,
R₃ für Wasserstoff, Methyl, Ethyl, Propyl, Hexyl, Heptyl, Octyl, 2-Methylpropyl, Nonyl, Decyl, Dodecyl, Hexadecyl, Octadecyl, Cyclopentyl, Cyclohexyl, Benzyl, Phenyl, Methoxy, Ethoxy, Propoxy, Butoxy, Pentoxy, Hexoxy, Heptoxy, Octoxy, 2-Ethylhexoxy, 1,3-Dimethylbutoxy, 1-Ethylpropoxy, 3,5,5-Trimethylhexoxy, Dodecyloxy, Hexadecyloxy, Octadecyloxy, Dimethylamino, Diethylamino, Dipropylamino, Dibutylamino, Diisopropylamino, Di(2-ethylbutyl)amino, Di(2-methylpropyl)amino, 1-Methylpropanamino, 1,1-Dimethylethanamino, Octylamino, Decylamino, Dodecylamino, Hexadecylamino, Octadecylamino, Cyclobutylamino, Cyclopentylamino, Cyclohexylamino steht,
R₄ für Wasserstoff, Methyl, Ethyl, Propyl, Butyl, Hexyl, Methoxy, Ethoxy, Propoxy, Butoxy, Hexoxy, Octoxy, Decyloxy, Dodecyloxy oder Chlor steht, und
R₅ für Wasserstoff, Methyl, Ethyl, Propyl, Butyl, Hexyl, Methoxy, Ethoxy, Propoxy, Butoxy, Hexoxy, Octoxy, Decyloxy, Dodecyloxy steht.

7. Carbonylaminostyryle des Anspruchs 1 der Formel 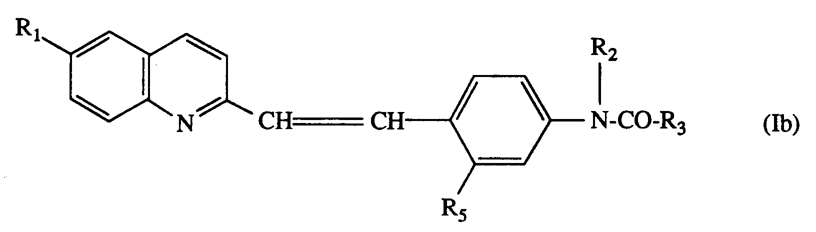 in welcher
R₁ für Wasserstoff, Methyl, Ethyl, Methoxy, Ethoxy oder Chlor steht,
R₂ für Wasserstoff, Methyl, Ethyl, n-Propyl, i-Propyl, n-Butyl, s-Butyl, i-Butyl oder t-Butyl steht,
R₃ für Wasserstoff, Methyl, Ethyl, Propyl, Hexyl, Heptyl, Octyl, 2-Methylpropyl, Nonyl, Decyl, Dodecyl, Hexadecyl, Octadecyl, Cyclopentyl, Cyclohexyl, Benzyl, Phenyl, Methoxy, Ethoxy, Propoxy, Butoxy, Pentoxy, Hexoxy, Heptoxy, Octoxy, 2-Ethylhexoxy, 1,3-Dimethylbutoxy, 1-Ethylpropoxy, 3,5,5-Trimethylhexoxy, Dodecyloxy, Hexadecyloxy, Octadecyloxy, Dimethylamino, Diethylamino, Dipropylamino, Dibutylamino, Diisopropylamino, Di(2-ethylbutyl)amino, Di(2-methylpropyl)amino, 1-Methylpropanamino, 1,1-Dimethylethanamino, Octylamino, Decylamino, Dodecylamino, Hexadecylamino, Octadecylamino, Cyclobutylamino, Cyclopentylamino, Cyclohexylamino steht,
R₅ für Wasserstoff, Methyl, Ethyl, Propyl, Butyl, Hexyl, Methoxy, Ethoxy, Propoxy, Butoxy, Hexoxy, Octoxy, Decyloxy, Dodecyloxy steht.

8. Verfahren zur Herstellung der Carbonylaminostyryle des Anspruchs 1, dadurch gekennzeichnet, daß
A) Verbindungen der Formel (II) 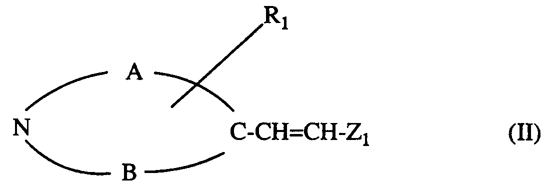 in welcher
A, B und R₁ die in Anspruch 1 angegebene Bedeutung haben, und
Z₁ für 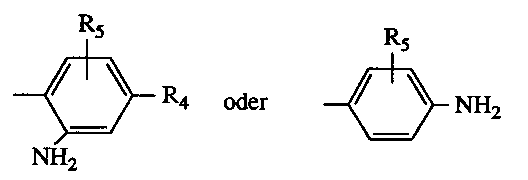 steht
worin R₄ und R₅ die in Anspruch 1 angegebene Bedeutung haben entweder
a) mit Verbindungen der Formel 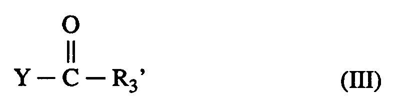 in welcher
Y für Halogen,
insbesondere für Chlor oder Brom steht, und
R₃' für Alkyl, Alkenyl, Cycloalkyl, Aryl, Aralkyl oder Hetaryl,
insbesondere für Alkyl mit 1 bis 18 Kohlenstoffatomen, das gegebenenfalls einfach oder mehrfach durch Alkoxy mit 1 bis 6 Kohlenstoffatomen, Cyano, Hydroxy oder Halogen substituiert ist, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Hydroxy oder Halogen substituiert ist, Aryl oder Aralkyl mit 1 bis 6 Kohlenstoffatomen im Alkylteil und jeweils 6 bis 10 Kohlenstoffatomen im Arylteil, welches jeweils gegebenenfalls im Arylteil einfach oder mehrfach durch Alkyl mit 1 bis 6 Kohlenstoffatomen, Hydroxy oder Halogen substituiert ist, Alkenyl mit 2 bis 6 Kohlenstoffatomen, oder für einen gesättigten oder ungesättigten 5- bis 7-gliedrigen heterocyclischen Ring, der 1 bis 4 Heteroatome wie Sauerstoff, Schwefel oder Stickstoff enthält und gegebenenfalls einfach oder mehrfach durch Alkyl mit 1 bis 6 Kohlenstoffatomen substiutiert ist, steht,
acyliert werden, oder
b) mit Chlorameisensäureestern zu den Verbindungen der Formel 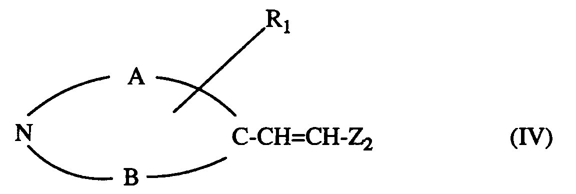 in welcher
Z₂ für 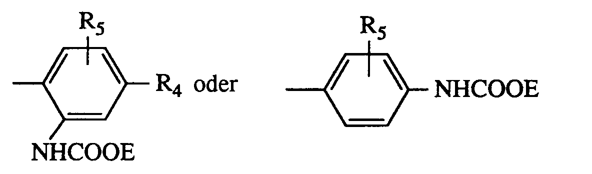 steht worin
R₄ und R₅ die in Anspruch 1 angegebene Bedeutung haben, und
E für Methyl, Ethyl oder Phenyl steht,
A, B und R₁ die in Anspruch 1 angegebene Bedeutung haben,
umgesetzt werden, und die so erhaltenen Verbindungen der Formel (IV) gegebenenfalls weiter mit
α) Aminen der Formel 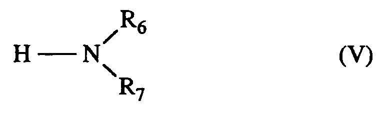 in welcher
R₆ und R₇ gleich oder verschieden sind und jeweils für Wasserstoff, Alkyl, Cycloalkyl, Aryl oder Aralkyl,
insbesondere für Wasserstoff, Alkyl mit 1 bis 18 Kohlenstoffatomen, das gegebenenfalls einfach oder mehrfach durch Alkoxy mit 1 bis 6 Kohlenstoffatomen, Cyano, Hydroxy oder Halogen substituiert ist, Cycloalkyl mit 3 bis 8 Kohlenstoffatomen, das gegebenenfalls einfach oder mehrfach durch Alkyl oder Alkoxy mit jeweils 1 bis 6 Kohlenstoffatomen, Hydroxy oder Halogen substituiert ist, Aryl oder Aralkyl mit 1 bis 6 Kohlenstoffatomen im Alkylteil und jeweils 6 bis 10 Kohlenstoffatomen im Arylteil, welches jeweils gegebenenfalls im Arylteil einfach oder mehrfach durch Alkyl mit 1 bis 6 Kohlenstoffatomen, Hydroxy oder Halogen substituiert ist, steht,
oder
β) Alkoholen der Formel
HO-R₈ (VI)
in welcher
R₈ für Alkyl
insbesondere für Alkyl mit 3 bis 18 Kohlenstoffatomen, das gegebenenfalls einfach oder mehrfach durch Alkoxy mit 1 bis 6 Kohlenstoffatomen, Cyano, Hydroxy oder Halogen substituiert ist, steht.
umgesetzt werden, oder
B) Verbindungen der Formel (VII) 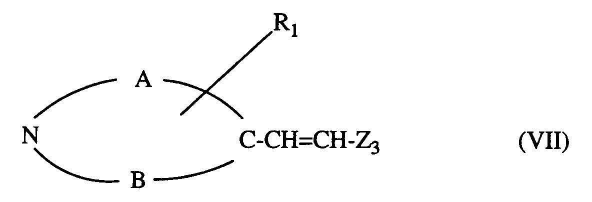 in welcher
A, B und R₁ die in Anspruch 1 angegebene Bedeutung haben, und
Z₃ für 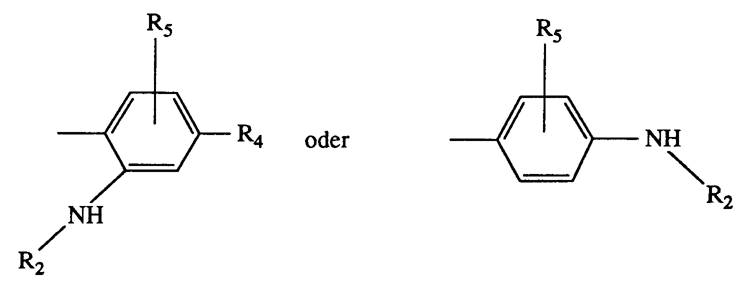 steht
worin
R₂, R₄ und R₅ die in Anspruch 1 angegebene Bedeutung haben
entweder
a) mit Verbindungen der Formel (III) umgesetzt werden, oder
b) mit Chlorameisensäureethylester zu den entsprechenden ethoxycarbonylamino-substituierten Verbindungen der Formel (I) umgesetzt werden, und diese Verbindung gegebenenfalls durch Umsetzung mit Aminen der Formel (V) oder Alkoholen der Formel (VI) wie unter (A-b(α)) und (A-b(β)) beschrieben weiter umgesetzt werden.

9. Verwendung der Carbonylaminostyryle der Ansprüche 1 bis 7 für druckkopierfähige, thermoreaktive oder elektrochrome Aufzeichnungsmaterialien, die außerdem einen sauren Farbentwickler enthalten.

10. Verwendung nach Anspruch 9, dadurch gekennzeichnet, daß man die Carbonylaminostyryle im Gemisch mit bekannten Farbbildnern verwendet.
